# EUROPEAN PATENT SPECIFICATION

(11) **EP 3 063 173 B1**
(45) Date of publication and mention of the grant of the patent: **29.07.2020**
(21) Application number: 14799089.9
(22) Date of filing: 31.10.2014
(51) Int. Cl.: C07K 16/28, A61K 39/395, A61P 35/02

(54) **SPECIFIC ANTI-CD38 ANTIBODIES FOR TREATING HUMAN CANCERS**
SPEZIFISCHE ANTIKÖRPER GEGEN CD38 ZUR BEHANDLUNG VON MENSCHLICHEM KREBS
ANTICORPS ANTI-CD38 SPÉCIFIQUES POUR LE TRAITEMENT DE CANCERS HUMAINS

(30) Priority: 31.10.2013 US 201361898309 P; 31.07.2014 EP 14306220
(43) Date of publication of application: 07.09.2016
(73) Proprietor: SANOFI, 75008 Paris (FR)
(72) Inventor: DESLANDES, Antoine, Bridgewater, NJ 08807 (US); GRZEGORZEWSKI, Krzysztof, J., Randolph, NJ 07869 (US); OZOUX, Marie-laure, Bridgewater, NJ 08807 (US); TOMKINSON, Blake, Weston, MA 02493 (US)
(74) Representative: Lavoix
(86) International application number: PCT/US2014/063380
(87) International publication number: WO 2015/066450

(56) References cited:
- WO-A2-2008/047242
- WO-A2-2013/059885
- CHILLEMI ANTONELLA ET AL: "Anti-CD38 antibody therapy: windows of opportunity yielded by the functional characteristics of the target molecule.", MOLECULAR MEDICINE (CAMBRIDGE, MASS.) 2013, vol. 19, May 2013 (2013-05), pages 99-108, XP002735274, ISSN: 1528-3658
- MARTIN THOMAS G III ET AL: "SAR650984, a CD38 Monoclonal Antibody In Patients With Selected CD38+Hematological Malignancies- Data From a Dose-Escalation Phase I Study", BLOOD, vol. 122, no. 21, November 2013 (2013-11), page 284, XP002735275, & 55TH ANNUAL MEETING OF THE AMERICAN-SOCIETY-OF-HEMATOLOGY; NEW ORLEANS, LA, USA; DECEMBER 07 -10, 2013
- MARTIN T ET AL: "A PHASE IB DOSE ESCALATION TRIAL OF SAR650984 (ANTI-CD-38 MAB) IN COMBINATION WITH LENALIDOMIDE AND DEXAMETHASONE IN RELAPSED/REFRACTORY MULTIPLE MYELOMA", HAEMATOLOGICA, vol. 99, no. Suppl. 1, June 2014 (2014-06) , page 114, XP002735276, & 19TH CONGRESS OF THE EUROPEAN-HEMATOLOGY-ASSOCIATION; MILAN, ITALY; JUNE 12 -15, 2014 ISSN: 0390-6078
- GOPALAKRISHNAN S ET AL: "Daratumumab improves the anti-myeloma effect of newly emerging multidrug therapies", BLOOD AND LYMPHATIC CANCER: TARGETS AND THERAPY 20130107 DOVE MEDICAL PRESS LTD. NZL, vol. 3, 7 January 2013 (2013-01-07), pages 19-24, XP002735277, ISSN: 1179-9889
- RICHARDSON P ET AL: "Daratumumab. Anti-CD38 monoclonal antibody, treatment of multiple myeloma", DRUGS OF THE FUTURE 2013 PROUS SCIENCE ESP, vol. 38, no. 8, August 2013 (2013-08), pages 545-554, XP002735278, ISSN: 0377-8282
- Sanofi: "Dose Escalation Study of Anti-CD38 Monoclonal Antibody in Patients With Selected CD38+ Hematological Malignancies", ClinicalTrials.gov arhivve , 15 October 2013 (2013-10-15), XP002735279, Retrieved from the Internet: URL:https://clinicaltrials.gov/archive/NCT 01084252/2013_10_15 [retrieved on 2015-01-30]
- Torben Plesner, Henk Lokhorst, Peter Gimsing, Hareth Nahi, Steen Lisby and Paul G. Richardson: "Daratumumab, a CD38 Monoclonal Antibody in Patients with Multiple Myeloma - Data From a Dose-Escalation Phase I/II Study", Blood - Journal , December 2012 (2012-12), Retrieved from the Internet: URL:http://www.bloodjournal.org/content/12 0/21/73 [retrieved on 2017-11-08]

### SEQUENCE LISTING

<110> Sanofi
<120> Specific anti-CD38 antibodies for treating human cancers
<130> 562803
<160> 63
<170> PatentIn version 3.5
<210> 1
   <211> 5
   <212> PRT
   <213> Mus sp.
<400> 1
   Ser Tyr Gly Met Asn
   1               5
<210> 2
   <211> 17
   <212> PRT
   <213> Mus sp.
<400> 2
   Trp Ile Asn Thr Tyr Thr Gly Glu Pro Thr Tyr Ala Asp Asp Phe Lys
   1               5                   10                  15
   Gly
<210> 3
   <211> 5
   <212> PRT
   <213> Mus sp.
<400> 3
   Arg Gly Phe Ala Tyr
   1               5
<210> 4
   <211> 15
   <212> PRT
   <213> Mus sp.
<400> 4
   Arg Ala Ser Glu Ser Val Glu Ile Tyr Gly Asn Gly Phe Met Asn
   1 5 10 15
<210> 5
   <211> 7
   <212> PRT
   <213> Mus sp.
<400> 5
   Arg Ala Ser Asn Leu Glu Ser
   1 5
<210> 6
   <211> 9
   <212> PRT
   <213> Mus sp.
<400> 6
   Gln Gln Ile Asn Glu Asp Pro Phe Thr
   1 5
<210> 7
   <211> 5
   <212> PRT
   <213> Mus sp.
<400> 7
   Asn Ser Gly Met Asn
   1 5
<210> 8
   <211> 17
   <212> PRT
   <213> Mus sp.
<400> 8
   Trp Ile Asn Thr Tyr Thr Gly Glu Pro Thr Tyr Ala Asp Asp Phe Lys
   1 5 10 15
   Gly
<210> 9
   <211> 5
   <212> PRT
   <213> Mus sp.
<400> 9
   Arg Gly Phe Val Tyr
   1 5
<210> 10
   <211> 15
   <212> PRT
   <213> Mus sp.
<400> 10
   Arg Ala Ser Glu Ser Val Ala Ile Tyr Gly Asn Ser Phe Leu Lys
   1 5 10 15
<210> 11
   <211> 7
   <212> PRT
   <213> Mus sp.
<400> 11
   Arg Ala Ser Asn Leu Glu Ser
   1 5
<210> 12
   <211> 9
   <212> PRT
   <213> Mus sp.
<400> 12
   Gln Gln Ile Asn Glu Asp Pro Tyr Thr
   1 5
<210> 13
   <211> 5
   <212> PRT
   <213> Mus sp.
<400> 13
   Asp Tyr Trp Met Gln
   1 5
<210> 14
   <211> 17
   <212> PRT
   <213> Mus sp.
<400> 14
   Thr Ile Tyr Pro Gly Asp Gly Asp Thr Gly Tyr Ala Gln Lys Phe Lys
   1 5 10 15
   Gly
<210> 15
   <211> 11
   <212> PRT
   <213> Mus sp.
<400> 15
   Gly Asp Tyr Tyr Gly Ser Asn Ser Leu Asp Tyr
   1 5 10
<210> 16
   <211> 11
   <212> PRT
   <213> Mus sp.
<400> 16
   Lys Ala Ser Gln Asp Val Ser Thr Val Val Ala
   1 5 10
<210> 17
   <211> 7
   <212> PRT
   <213> Mus sp.
<400> 17
   Ser Ala Ser Tyr Arg Tyr Ile
   1 5
<210> 18
   <211> 9
   <212> PRT
   <213> Mus sp.
<400> 18
   Gln Gln His Tyr Ser Pro Pro Tyr Thr
   1 5
<210> 19
   <211> 5
   <212> PRT
   <213> Mus sp.
<400> 19
   Gly Ser Trp Met Asn
   1 5
<210> 20
   <211> 17
   <212> PRT
   <213> Mus sp.
<400> 20
   Arg Ile Tyr Pro Gly Asp Gly Asp Ile Ile Tyr Asn Gly Asn Phe Arg
   1 5 10 15
   Asp
<210> 21
   <211> 10
   <212> PRT
   <213> Mus sp.
<400> 21
   Trp Gly Thr Phe Thr Pro Ser Phe Asp Tyr
   1               5                   10
<210> 22
   <211> 11
   <212> PRT
   <213> Mus sp.
<400> 22
   Lys Ala Ser Gln Asp Val Val Thr Ala Val Ala
   1               5                   10
<210> 23
   <211> 7
   <212> PRT
   <213> Mus sp.
<400> 23
   Ser Ala Ser His Arg Tyr Thr
   1               5
<210> 24
   <211> 9
   <212> PRT
   <213> Mus sp.
<400> 24
   Gln Gln His Tyr Thr Thr Pro Thr Thr
   1               5
<210> 25
   <211> 5
   <212> PRT
   <213> Mus sp.
<400> 25
   Ser Tyr Thr Leu Ser
   1               5
<210> 26
   <211> 17
   <212> PRT
   <213> Mus sp.
<400> 26
   Thr Ile Ser Ile Gly Gly Arg Tyr Thr Tyr Tyr Pro Asp Ser Val Glu
   1               5                   10                  15
   Gly
<210> 27
   <211> 8
   <212> PRT
   <213> Mus sp.
<400> 27
   Asp Phe Asn Gly Tyr Ser Asp Phe
   1 5
<210> 28
   <211> 11
   <212> PRT
   <213> Mus sp.
<400> 28
   Lys Ala Ser Gln Val Val Gly Ser Ala Val Ala
   1 5 10
<210> 29
   <211> 7
   <212> PRT
   <213> Mus sp.
<400> 29
   Trp Ala Ser Thr Arg His Thr
   1 5
<210> 30
   <211> 9
   <212> PRT
   <213> Mus sp.
<400> 30
   Gln Gln Tyr Asn Ser Tyr Pro Tyr Thr
   1 5
<210> 31
   <211> 5
   <212> PRT
   <213> Mus sp.
<400> 31
   Asn Phe Gly Met His
   1 5
<210> 32
   <211> 17
   <212> PRT
   <213> Mus sp.
<400> 32
   Tyr Ile Arg Ser Gly Ser Gly Thr Ile Tyr Tyr Ser Asp Thr Val Lys
   1               5                   10                  15
   Gly
<210> 33
   <211> 11
   <212> PRT
   <213> Mus sp.
<400> 33
   Ser Tyr Tyr Asp Phe Gly Ala Trp Phe Ala Tyr
   1               5                   10
<210> 34
   <211> 11
   <212> PRT
   <213> Mus sp.
<400> 34
   Lys Ala Ser Gln Asn Val Gly Thr Asn Val Ala
   1               5                   10
<210> 35
   <211> 7
   <212> PRT
   <213> Mus sp.
<400> 35
   Ser Ala Ser Ser Arg Tyr Ser
   1               5
<210> 36
   <211> 9
   <212> PRT
   <213> Mus sp.
<400> 36
   Gln Gln Tyr Asn Ser Tyr Pro Leu Thr
   1               5
<210> 37
   <211> 17
   <212> PRT
   <213> Mus sp.
<400> 37
   Thr Ile Tyr Pro Gly Asp Gly Asp Thr Gly Tyr Ala Gln Lys Phe Gln
   1 5 10 15
   Gly
<210> 38
   <211> 17
   <212> PRT
   <213> Mus sp.
<400> 38
<210> 39
   <211> 112
   <212> PRT
   <213> Mus sp.
<400> 39
   Asp Ile Val Leu Thr Gln Ser Pro Ala Ser Leu Ala Val Ser Leu Gly
   1 5 10 15
   Gln Arg Ala Thr Ile Ser Cys Arg Ala Ser Glu Ser Val Ala Ile Tyr
   20 25 30
   Gly Asn Ser Phe Leu Lys Trp Phe Gln Gln Lys Pro Gly Gln Pro Pro
   35 40 45
   Lys Leu Leu Ile Tyr Arg Ala Ser Asn Leu Glu Ser Gly Ile Pro Ala
   50 55 60
   Arg Phe Ser Gly Ser Gly Ser Gly Thr Asp Phe Thr Leu Thr Ile Asn
   65 70 75 80
   Pro Val Glu Ala Asp Asp Val Ala Thr Tyr Tyr Cys Gln Gln Ile Asn
   85 90 95
   Glu Asp Pro Tyr Thr Phe Gly Gly Gly Thr Lys Leu Glu Ile Lys Arg
   100 105 110
<210> 40
   <211> 108
   <212> PRT
   <213> Mus sp.
<400> 40
   Asp Ile Val Met Ala Gln Ser His Lys Phe Met Ser Thr Ser Val Gly
   1 5 10 15
   Asp Arg Val Ser Ile Thr Cys Lys Ala Ser Gln Asp Val Ser Thr Val
   20 25 30
   Val Ala Trp Tyr Gln Gln Lys Pro Gly Gln Ser Pro Lys Arg Leu Ile
   35 40 45
   Tyr Ser Ala Ser Tyr Arg Tyr Ile Gly Val Pro Asp Arg Phe Thr Gly
   50 55 60
   Ser Gly Ser Gly Thr Asp Phe Thr Phe Thr Ile Ser Ser Val Gln Ala
   65 70 75 80
   Glu Asp Leu Ala Val Tyr Tyr Cys Gln Gln His Tyr Ser Pro Pro Tyr
   85 90 95
   Thr Phe Gly Gly Gly Thr Lys Leu Glu Ile Lys Arg
   100 105
<210> 41
   <211> 108
   <212> PRT
   <213> Mus sp.
<400> 41
   Asp Ile Val Met Thr Gln Ser His Lys Phe Leu Ser Thr Ser Val Gly
   1               5                   10                  15
   Asp Arg Val Ser Ile Thr Cys Lys Ala Ser Gln Asp Val Val Thr Ala
               20                  25                  30
   Val Ala Trp Phe Gln Gln Lys Pro Gly Gln Ser Pro Lys Leu Leu Ile
           35                  40                  45
   Tyr Ser Ala Ser His Arg Tyr Thr Gly Val Pro Asp Arg Phe Thr Gly
       50                  55                  60
   Ser Gly Ser Gly Thr Asp Phe Thr Phe Thr Ile Ile Ser Val Gln Ala
   65                  70                  75                  80
   Glu Asp Leu Ala Val Tyr Tyr Cys Gln Gln His Tyr Thr Thr Pro Thr
                   85                  90                  95
   Thr Phe Gly Gly Gly Thr Lys Leu Asp Phe Arg Arg
               100                 105
<210> 42
   <211> 108
   <212> PRT
   <213> Mus sp.
<400> 42
   Asp Thr Val Met Thr Gln Ser His Lys Phe Ile Ser Thr Ser Val Gly
   1               5                   10                  15
   Asp Arg Val Ser Ile Thr Cys Lys Ala Ser Gln Val Val Gly Ser Ala
               20                  25                  30
   Val Ala Trp Tyr Gln Gln Lys Pro Gly Gln Ser Pro Lys Leu Leu Ile
           35                  40                  45
   Tyr Trp Ala Ser Thr Arg His Thr Gly Val Pro Asp Arg Phe Thr Gly
       50                  55                  60
   Ser Gly Ser Gly Thr Asp Phe Thr Leu Thr Ile Ser Asn Val Gln Ser
   65                  70                  75                  80
   Glu Asp Leu Ala Asp Tyr Phe Cys Gln Gln Tyr Asn Ser Tyr Pro Tyr
                   85                  90                  95
   Thr Phe Gly Gly Gly Thr Lys Leu Glu Ile Lys Arg
               100                 105
<210> 43
   <211> 108
   <212> PRT
   <213> Mus sp.
<400> 43
   Asp Ile Val Met Thr Gln Ser Gln Lys Phe Met Ser Thr Ser Val Gly
   1               5                   10                  15
   Asp Arg Val Ser Val Thr Cys Lys Ala Ser Gln Asn Val Gly Thr Asn
               20                  25                  30
   Val Ala Trp Tyr Gln His Lys Pro Gly Gln Ser Pro Lys Ile Met Ile
           35                  40                  45
   Tyr Ser Ala Ser Ser Arg Tyr Ser Gly Val Pro Asp Arg Phe Thr Gly
       50                  55                  60
   Ser Gly Ser Gly Thr Leu Phe Thr Leu Thr Ile Asn Asn Val Gln Ser
   65                  70                  75                  80
   Glu Asp Leu Ala Glu Tyr Phe Cys Gln Gln Tyr Asn Ser Tyr Pro Leu
                   85                  90                  95
   Thr Phe Gly Ser Gly Thr Lys Leu Glu Ile Lys Arg
               100                 105
<210> 44
   <211> 114
   <212> PRT
   <213> Mus sp.
<400> 44
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Leu Thr Ser Tyr
               20                  25                  30
   Gly Met Asn Trp Val Lys Gln Ala Pro Gly Lys Gly Leu Lys Trp Met
           35                  40                  45
   Gly Trp Ile Asn Thr Tyr Thr Gly Glu Pro Thr Tyr Ala Asp Asp Phe
       50                  55                  60
   Lys Gly Arg Phe Ala Phe Ser Leu Glu Thr Ser Ala Ser Thr Ala Phe
   65                  70                  75                  80
   Leu Gln Ile Asn Asn Leu Lys Asn Glu Asp Thr Ala Thr Tyr Phe Cys
                   85                  90                  95
   Val Arg Arg Gly Phe Ala Tyr Trp Gly Gln Gly Thr Leu Val Thr Val
               100                 105                 110
   Ser Ala
<210> 45
   <211> 114
   <212> PRT
   <213> Mus sp.
<400> 45
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Asn Ser
               20                  25                  30
   Gly Met Asn Trp Val Lys Gln Ala Pro Gly Lys Gly Leu Lys Trp Met
           35                  40                  45
   Gly Trp Ile Asn Thr Tyr Thr Gly Glu Pro Thr Tyr Ala Asp Asp Phe
       50                  55                  60
   Lys Gly Arg Phe Ala Phe Ser Leu Glu Thr Ser Ala Ser Ser Ala Tyr
   65                  70                  75                  80
   Leu Gln Ile Ser Asn Leu Lys Asn Glu Asp Thr Ala Thr Tyr Phe Cys
                   85                  90                  95
   Ala Arg Arg Gly Phe Val Tyr Trp Gly Gln Gly Thr Leu Val Thr Val
               100                 105                 110
   Ser Ala
<210> 46
   <211> 120
   <212> PRT
   <213> Mus sp.
<400> 46
   Gln Val Gln Leu Gln Gln Ser Gly Ala Glu Leu Ala Arg Pro Gly Thr
   1               5                   10                  15
   Ser Val Lys Leu Ser Cys Lys Ala Ser Gly Tyr Thr Phe Thr Asp Tyr
               20                  25                  30
   Trp Met Gln Trp Val Lys Gln Arg Pro Gly Gln Gly Leu Glu Trp Ile
   35 40 45
   Gly Thr Ile Tyr Pro Gly Asp Gly Asp Thr Gly Tyr Ala Gln Lys Phe
   50 55 60
   Lys Gly Lys Ala Thr Leu Thr Ala Asp Lys Ser Ser Lys Thr Val Tyr
   65 70 75 80
   Met His Leu Ser Ser Leu Ala Ser Glu Asp Ser Ala Val Tyr Tyr Cys
   85 90 95
   Ala Arg Gly Asp Tyr Tyr Gly Ser Asn Ser Leu Asp Tyr Trp Gly Gln
   100 105 110
   Gly Thr Ser Val Thr Val Ser Ser
   115 120
<210> 47
   <211> 119
   <212> PRT
   <213> Mus sp.
<400> 47
   Gln Val Gln Leu Gln Gln Ser Gly Pro Glu Leu Val Arg Pro Gly Ala
   1 5 10 15
   Ser Val Lys Ile Ser Cys Lys Thr Ser Gly Tyr Ala Phe Ser Gly Ser
   20 25 30
   Trp Met Asn Trp Val Lys Gln Arg Pro Gly Gln Gly Leu Glu Trp Ile
   35 40 45
   Gly Arg Ile Tyr Pro Gly Asp Gly Asp Ile Ile Tyr Asn Gly Asn Phe
   50 55 60
   Arg Asp Lys Val Thr Leu Ser Ala Asp Lys Ser Ser Asn Thr Ala Tyr
   65 70 75 80
   Met Gln Leu Ser Ser Leu Thr Ser Val Asp Ser Ala Val Tyr Phe Cys
   85 90 95
   Ser Arg Trp Gly Thr Phe Thr Pro Ser Phe Asp Tyr Trp Gly Gln Gly
   100 105 110
   Thr Thr Leu Thr Val Ser Ser
   115
<210> 48
   <211> 117
   <212> PRT
   <213> Mus sp.
<400> 48
   Asp Val Lys Leu Val Glu Ser Gly Gly Gly Leu Val Lys Pro Gly Gly
   1               5                   10                  15
   Ser Leu Lys Leu Ser Cys Glu Ala Ser Gly Phe Thr Phe Ser Ser Tyr
               20                  25                  30
   Thr Leu Ser Trp Val Arg Gln Thr Pro Glu Thr Arg Leu Glu Trp Val
           35                  40                  45
   Ala Thr Ile Ser Ile Gly Gly Arg Tyr Thr Tyr Tyr Pro Asp Ser Val
       50                  55                  60
   Glu Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Met Tyr Tyr Cys
                   85                  90                  95
   Thr Arg Asp Phe Asn Gly Tyr Ser Asp Phe Trp Gly Gln Gly Thr Thr
               100                 105                 110
   Leu Thr Val Ser Ser
           115
<210> 49
   <211> 120
   <212> PRT
   <213> Mus sp.
<400> 49
   Asn Val Gln Leu Val Glu Ser Gly Gly Gly Leu Val Gln Pro Gly Gly
   1               5                   10                  15
   Ser Arg Lys Leu Ser Cys Ala Ala Ser Gly Phe Thr Phe Ser Asn Phe
               20                  25                  30
   Gly Met His Trp Val Arg Gln Ala Pro Glu Lys Gly Leu Glu Trp Val
           35                  40                  45
   Ala Tyr Ile Arg Ser Gly Ser Gly Thr Ile Tyr Tyr Ser Asp Thr Val
   50 55 60
   Lys Gly Arg Phe Thr Ile Ser Arg Asp Asn Pro Lys Asn Thr Leu Phe
   65 70 75 80
   Leu Gln Met Thr Ser Leu Arg Ser Glu Asp Thr Ala Met Tyr Tyr Cys
   85 90 95
   Ala Arg Ser Tyr Tyr Asp Phe Gly Ala Trp Phe Ala Tyr Trp Gly Gln
   100 105 110
   Gly Thr Leu Val Thr Val Ser Ala
   115 120
<210> 50
   <211> 120
   <212> PRT
   <213> Homo sapiens
<400> 50
<210> 51
   <211> 117
   <212> PRT
   <213> Homo sapiens
<400> 51
   Glu Val Gln Leu Val Glu Ser Gly Gly Gly Leu Val Lys Pro Gly Gly
   1               5                   10                  15
   Ser Leu Arg Leu Ser Cys Glu Ala Ser Gly Phe Thr Phe Ser Ser Tyr
               20                  25                  30
   Thr Leu Ser Trp Val Arg Gln Thr Pro Gly Lys Gly Leu Glu Trp Val
           35                  40                  45
   Ala Thr Ile Ser Ile Gly Gly Arg Tyr Thr Tyr Tyr Pro Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Lys Ser Glu Asp Thr Ala Met Tyr Tyr Cys
                   85                  90                  95
   Thr Arg Asp Phe Asn Gly Tyr Ser Asp Phe Trp Gly Gln Gly Thr Thr
               100                 105                 110
   Leu Thr Val Ser Ser
           115
<210> 52
   <211> 108
   <212> PRT
   <213> Homo sapiens
<400> 52
   Asp Ile Val Met Thr Gln Ser His Leu Ser Met Ser Thr Ser Leu Gly
   1               5                   10                  15
   Asp Pro Val Ser Ile Thr Cys Lys Ala Ser Gln Asp Val Ser Thr Val
               20                  25                  30
   Val Ala Trp Tyr Gln Gln Lys Pro Gly Gln Ser Pro Arg Arg Leu Ile
           35                  40                  45
   Tyr Ser Ala Ser Tyr Arg Tyr Ile Gly Val Pro Asp Arg Phe Thr Gly
       50                  55                  60
   Ser Gly Ala Gly Thr Asp Phe Thr Phe Thr Ile Ser Ser Val Gln Ala
   65                  70                  75                  80
   Glu Asp Leu Ala Val Tyr Tyr Cys Gln Gln His Tyr Ser Pro Pro Tyr
                   85                  90                  95
   Thr Phe Gly Gly Gly Thr Lys Leu Glu Ile Lys Arg
               100                 105
<210> 53
   <211> 108
   <212> PRT
   <213> Homo sapiens
<400> 53
   Asp Ile Val Met Ala Gln Ser His Leu Ser Met Ser Thr Ser Leu Gly
   1               5                   10                  15
   Asp Pro Val Ser Ile Thr Cys Lys Ala Ser Gln Asp Val Ser Thr Val
               20                  25                  30
   Val Ala Trp Tyr Gln Gln Lys Pro Gly Gln Ser Pro Arg Arg Leu Ile
           35                  40                  45
   Tyr Ser Ala Ser Tyr Arg Tyr Ile Gly Val Pro Asp Arg Phe Thr Gly
       50                  55                  60
   Ser Gly Ala Gly Thr Asp Phe Thr Phe Thr Ile Ser Ser Val Gln Ala
   65                  70                  75                  80
   Glu Asp Leu Ala Val Tyr Tyr Cys Gln Gln His Tyr Ser Pro Pro Tyr
                   85                  90                  95
   Thr Phe Gly Gly Gly Thr Lys Leu Glu Ile Lys Arg
               100                 105
<210> 54
   <211> 108
   <212> PRT
   <213> Homo sapiens
<400> 54
   Asp Thr Val Met Thr Gln Ser Pro Ser Thr Ile Ser Thr Ser Val Gly
   1               5                   10                  15
   Asp Arg Val Ser Ile Thr Cys Lys Ala Ser Gln Val Val Gly Ser Ala
               20                  25                  30
   Val Ala Trp Tyr Gln Gln Lys Pro Gly Gln Ser Pro Lys Leu Leu Ile
           35                  40                  45
   Tyr Trp Ala Ser Thr Arg His Thr Gly Val Pro Asp Arg Phe Thr Gly
       50                  55                  60
   Ser Gly Ser Gly Thr Asp Phe Thr Leu Thr Ile Ser Asn Val Gln Ser
   65                  70                  75                  80
   Asp Asp Leu Ala Asp Tyr Phe Cys Gln Gln Tyr Asn Ser Tyr Pro Tyr
                   85                  90                  95
   Thr Phe Gly Gly Gly Thr Lys Leu Glu Ile Lys Arg
               100                 105
<210> 55
   <211> 108
   <212> PRT
   <213> Homo sapiens
<400> 55
<210> 56
   <211> 36
   <212> DNA
   <213> Artificial Sequence
<220>
   <223> Primer
<400> 56
   ggaggatcca tagacagatg ggggtgtcgt tttggc 36
<210> 57
   <211> 32
   <212> DNA
   <213> Artificial Sequence
<220>
   <223> Primer
<400> 57
   ggaggatccc ttgaccaggc atcctagagt ca 32
<210> 58
   <211> 32
   <212> DNA
   <213> Artificial Sequence
<220>
   <223> Primer
<220>
   <221> misc_feature
   <222> (13)..(13)
   <223> s is g or c
<220>
   <221> misc_feature
   <222> (15)..(15)
   <223> r is a or g
<220>
   <221> misc_feature
   <222> (18)..(18)
   <223> n is a, c, g, or t
<220>
   <221> misc_feature
   <222> (19)..(19)
   <223> m is a or c
<220>
   <221> misc_feature
   <222> (25)..(25)
   <223> s is g or c
<220>
   <221> misc_feature
   <222> (28)..(28)
   <223> s is g or c
<400> 58
   cttccggaat tcsargtnma gctgsagsag tc 32
<210> 59
   <211> 35
   <212> DNA
   <213> Artificial Sequence
<220>
   <223> Primer
<220>
   <221> misc_feature
   <222> (13)..(13)
   <223> s is g or c
<220>
   <221> misc_feature
   <222> (15)..(15)
   <223> r is a or g
<220>
   <221> misc_feature
   <222> (18)..(18)
   <223> n is a, c, g, or t
<220>
   <221> misc_feature
   <222> (19)..(19)
   <223> m is a or c
<220>
   <221> misc_feature
   <222> (25)..(25)
   <223> s is g or c
<220>
   <221> misc_feature
   <222> (28)..(28)
   <223> s is g or c
<220>
   <221> misc_feature
   <222> (33)..(33)
   <223> w is a or t
<400> 59
   cttccggaat tcsargtnma gctgsagsag tcwgg 35
<210> 60
   <211> 31
   <212> DNA
   <213> Artificial Sequence
<220>
   <223> Primer
<220>
   <221> misc_feature
   <222> (10)..(10)
   <223> y is a or g
<220>
   <221> misc_feature
   <222> (17)..(17)
   <223> m is a or c
<220>
   <221> misc_feature
   <222> (19)..(19)
   <223> s is g or c
<220>
   <221> misc_feature
   <222> (22)..(22)
   <223> m is a or c
<220>
   <221> misc_feature
   <222> (25)..(25)
   <223> r is a or g
<220>
   <221> misc_feature
   <222> (26).. (26)
   <223> w is a or t
<220>
   <221> misc_feature
   <222> (29).. (29)
   <223> m is a or c
<400> 60
   ggagctcgay attgtgmtsa cmcarwctmc a 31
<210> 61
   <211> 46
   <212> DNA
   <213> Artificial Sequence
<220>
   <223> Primer
<400> 61
   tatagagctc aagcttggat ggtgggaaga tggatacagt tggtgc 46
<210> 62
   <211> 21
   <212> DNA
   <213> Artificial Sequence
<220>
   <223> Primer
<400> 62
   atggagtcac agattcaggt c 21
<210> 63
   <211> 32
   <212> DNA
   <213> Artificial Sequence
<220>
   <223> Primer
<400> 63
   ttttgaattc cagtaacttc aggtgtccac tc 32

## Description

### Field of the Invention

The present disclosure concerns treatment of disease using antibodies. More specifically, it relates to the use of anti-CD38 antibodies as a medicament or in the making of a medicament for treating cancers, such as multiple myeloma.

### Background

CD38 is a 45 kD type II transmembrane glycoprotein with a long C-terminal extracellular domain and a short N-terminal cytoplasmic domain. The CD38 protein is a bifunctional ectoenzyme that can catalyze the conversion of NAD+ into cyclic ADP-ribose (cADPR) and also hydrolyze cADPR into ADP-ribose.

CD38 is upregulated in many hematological malignancies and in cell lines derived from various hematological malignancies. Furthermore, most primitive pluripotent stem cells of the hematological system are CD38⁻. CD38 expression in hematological malignancies and its correlation with disease progression in chronic lymphocytic leukemia (CLL) makes CD38 an attractive target for antibody therapy.

Anti-CD38 antibodies, which specifically recognize CD38, have been previously described, for example in the international patent application WO2006/099875. However, these antibodies fail to induce apoptosis when used as a single agent and incubated with CD38⁺ expressing cells.

The specific monoclonal anti-CD38 antibodies, such as 38SB13, 38SB18, 38SB19, 38SB30, 38SB31 and 38SB39, have been previously described in international patent application WO2008/047242. WO2008/047242 describes the three cytotoxic activities, apoptosis, ADCC and CDC of these specific anti-CD38 antibodies.

Furthermore, the use of these specific anti-CD38⁺ antibodies in combination with cytotoxic agents, such as, cytarabine, vincristine, cyclophosphomide and melphalan has been reported in international patent applications WO2010/061357, WO2010/061358, WO2010/061359 and WO2010/061360. However, the use of anti-CD38 antibodies as a single agent has not been reported.

### Summary of the invention

In one embodiment, the present disclosure relates to an antibody that specifically binds CD38 for use as a medicament, wherein the antibody is to be administered to a human subject in a safe therapeutic dose of 20 mg/kg or below.

In another aspect, the safe therapeutic dose of the disclosed antibody is about 5 mg/kg, or about 10 mg/kg, or about 20 mg/kg.

In another embodiment, the present disclosure also relates to an antibody that specifically binds CD38 or a pharmaceutical composition comprising an antibody that specifically binds CD38, wherein the antibody or the pharmaceutical composition may be used as a medicament in the treatment of relapsed and/or refractory multiple myeloma, wherein the antibody is to be administered to a human subject in a safe therapeutic dose of about 20 mg/kg or less.

In another embodiment, the present disclosure also relates to the use of anti-CD38 antibodies as a single agent which reduces the patients' charge with chemotherapeutics.

In another embodiment, the present disclosure further relates to a safe therapeutic dose to a human subject of an antibody that specifically binds CD38, wherein said safe therapeutic dose is 20 mg/kg or below, or about 20 mg/kg or below.

In another embodiment, the disclosure also concerns a method of treating a CD38⁺ hematological malignancy in a human subject in need thereof, said method comprising administering to said human subject an antibody that specifically binds CD38 at a safe therapeutically effective amount of 20 mg/kg or below, or about 20 mg/kg or below.

In another aspect, the disclosure also concerns a method of treating CD38⁺ multiple myeloma in a human subject in need thereof, said method comprising administering to said human subject an effective amount of an antibody that specifically binds CD38.

In another aspect, the disclosure also concerns the use of said antibody that specifically binds CD38 for the manufacture of a medicament, wherein said antibody is administered to a human subject in a safe therapeutic dose of 20 mg/kg or below, or about 20 mg/kg or below.

In one embodiment, the medicament is for the treatment of a CD38⁺ hematological malignancy, in particular multiple Myeloma, most particularly relapsed and/or refractory CD38⁺ multiple Myeloma.

In another embodiment, the present disclosure further concerns the use of said antibody that specifically binds CD38 for the manufacture of a medicament for the treatment of CD38⁺ multiple Myeloma, in particular relapsed and/or refractory CD38⁺ multiple Myeloma.

In one embodiment, an antibody or epitope binding fragment thereof capable of specifically binding CD38 is disclosed for use as a medicament, wherein the antibody or epitope binding fragment does not trigger production of autoantibodies against said antibody or epitope binding fragment by a human subject when the antibody or epitope binding fragment is administered to the human subject at a dose of about 20 mg/kg or less.

In another embodiment, an antibody or epitope binding fragment thereof capable of specifically binding CD38 is disclosed for use as a medicament, wherein the antibody or epitope binding fragment is capable of exhibiting detectable CD38 receptor occupancy in a human subject when administered to the human subject at a dose level of about 1 mg/kg every two weeks.

In another embodiment, an antibody or epitope binding fragment thereof capable of specifically binding CD38 is disclosed for use as a medicament, wherein the antibody or epitope binding fragment is capable of exhibiting at least about 84.1% CD38 receptor occupancy in a human subject when administered to the human subject a dose level of about 10 mg/kg every two weeks

In another embodiment, an antibody or epitope binding fragment thereof capable of specifically binding CD38 is disclosed for use as a medicament, wherein the antibody or epitope binding fragment is capable of exhibiting at least about 97.7% CD38 receptor occupancy in a human subject when administered to the human subject a dose level of about 10 mg/kg every two weeks.

In another aspect, an antibody or epitope binding fragment thereof capable of specifically binding CD38 is disclosed for use as a medicament, wherein the antibody or epitope binding fragment is capable of inhibiting tumor growth in a human subject when administered to the human subject at a dose level ranging from about 5 mg/kg to about 20 mg/kg every two weeks, or every week.

In another embodiment, an antibody or epitope binding fragment thereof capable of specifically binding CD38 is disclosed for use as a medicament, wherein the antibody or epitope binding fragment is capable of inhibiting tumor growth in a human subject when administered to the human subject at a dose level ranging from about 10 mg/kg to about 20 mg/kg every two weeks, or every week.

In another aspect, a pharmaceutical composition comprising any of the disclosed antibodies and a pharmaceutically acceptable carrier or excipient is also disclosed.

In another aspect, the present disclosure provides a unit dosage form comprising the pharmaceutical composition disclosed herein.

In another aspect, the present disclosure also provides an article of manufacture comprising the pharmaceutical composition disclosed herein and a container.

In another aspect, the disclosed methods may be suitable for treating a human subject having a disease or a disorder in which CD38 is abnormally upregulated. Such methods may include, among other steps, administering to the human subject an antibody or epitope binding fragment thereof capable of specifically binding CD38, wherein the antibody is capable of inhibiting tumor growth in the human subject when administered to the human subject at a dose level ranging from about 1 mg/kg to about 20 mg/kg every two weeks, or every week. In one aspect, such disease or disorder is a CD38⁺ hematological malignancy. In another aspect, such disease or disorder is a CD38⁺ multiple myeloma.

The invention is defined by the claims.

### BRIEF DESCRIPTION OF THE DRAWINGS

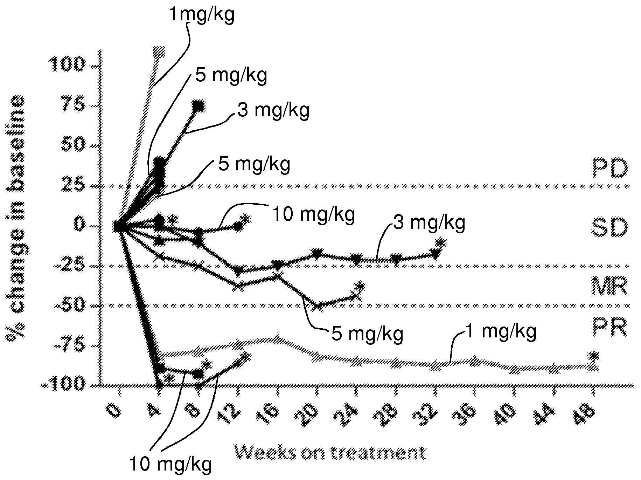

**Figure 1****:** Graph showing the response over time in patients with multiple myeloma (N=17) that were treated with the specific anti-CD38 antibody hu38SB19 at different doses (1, 3, 5 and 10 mg/kg). (Some patients were excluded after a short period of treatment due to a progressive disease. However, several patients are stabilized during the treatment and others at dose levels of 1 mg/kg and 10 mg/kg show a partial response. PR= partial response, MR= minimal response, SD= stable disease or PD=progressive disease).

### DETAILED DESCRIPTION

In the context of the disclosure the term "CD38" refers to a CD38 protein which is a 45 kD type II transmembrane glycoprotein with a long C-terminal extracellular domain and a short N-terminal cytoplasmic domain. The CD38 protein is a bifunctional ectoenzyme that can catalyze the conversion of NAD+ into cyclic ADP-ribose (cADPR) and also hydrolyze cADPR into ADP-ribose. During ontogeny, CD38 appears on CD34+ committed stem cells and lineage-committed progenitors of lymphoid, erythroid and myeloid cells. CD38 expression persists mostly in the lymphoid lineage with varying expression levels at different stages of T and B cell development.

The role of CD38⁺ in signal transduction has been further described in the international patent application WO2008/047242. In particular, CD38 refers to a type II transmembrane protein, comprising, for example, an amino acid sequence as in Genbank accession number NP_001766.2 (as available on October 7, 2013).

CD38 is upregulated in many hematological malignancies and in cell lines derived from various hematological malignancies.

"Hematological malignancies" are the types of cancer that affect blood, bone marrow, and lymph nodes. As the three are intimately connected through the immune system, a disease affecting one of the three might affect the others as well. Hematological malignancies include non-Hodgkin's lymphoma (NHL) (including, e.g. Burkitt's lymphoma (BL) and T cell lymphoma (TCL)), multiple myeloma (MM), chronic lymphocytic leukemia (CLL) (such as e.g. B chronic lymphocytic leukemia (B-CLL) and hairy cell leukemia (HCL)), B and T acute lymphocytic leukemia (ALL), acute myeloid leukemia (AML), Hodgkin's Lymphoma (HL), and chronic myeloid leukemia (CML).

On the other hand, most primitive pluripotent stem cells of the hematological system are CD38⁻. CD38 expression in hematological malignancies and its correlation with disease progression makes CD38 an attractive target for antibody therapy.

A "CD38⁺ cell" is a cell expressing the CD38 protein. In particular, the CD38⁺ cell is a mammalian cell. In one embodiment the CD38⁺ cell is a non-Hodgkin's lymphoma (NHL), multiple myeloma (MM), chronic lymphocytic leukemia (CLL), B and T acute lymphocytic leukemia (ALL), acute myeloid leukemia (AML), Hodgkin's Lymphoma (HL), or chronic myeloid leukemia (CML) cell expressing the CD38 protein.

"CD38⁺ hematological malignancy" is thus a hematological malignancy, as described above, wherein the cancerous cells are or comprise CD38⁺ cells.

In the context of the disclosure the CD38⁺ hematological malignancy is in particular selected from the group consisting of non-Hodgkin's lymphoma (NHL) (including, e.g. Burkitt's lymphoma (BL) and T cell lymphoma (TCL)), multiple myeloma (MM), chronic lymphocytic leukemia (CLL) (such as e.g. B chronic lymphocytic leukemia (B-CLL) or hairy cell leukemia (HCL)), B and T acute lymphocytic leukemia (ALL), acute myeloid leukemia (AML), Hodgkin's Lymphoma (HL), and chronic myeloid leukemia (CML), wherein the cancerous cells are or comprise CD38⁺ cells.

In particular, CD38⁺ hematological malignancies are B-cell non-Hodgkins Lymphoma (NHL), multiple myeloma (MM), acute myeloid leukaemia (AML), acute lymphoblastic leukaemia (B-cell ALL) and/or chronic lymphocytic leukaemia (CLL), more particularly multiple myeloma (MM), most particularly relapsed and/or refractory multiple Myeloma.

Methods to identify a hematological malignancy are known to the skilled in the art and include as a first step a complete blood count (CBC) and a test of the peripheral blood smear. Definitive diagnosis usually requires an adequate bone marrow aspiration and/or biopsy for morphology studies eventually complemented by flow cytometry analysis, cytogenetics and further molecular techniques.

Techniques to confirm that the cells derived from this hematological malignancy are CD38⁺ are known to the skilled in the art and include standard molecular biology techniques such as, for example, polymerase chain reaction (PCR) and/or immunochemical methods such as Western Blot analysis.

In the context of the disclosure, "subject" denotes a human.

In particular, "subject" and/or "subject in need thereof" denotes herein an individual that is affected with a CD38⁺ hematological malignancy and under medical care or treatment. The word subject may thus refer herein to a patient.

The subject according to the disclosure may be a male or a female.

In some embodiments, the subject has been previously treated with an anti-cancer therapy. In particular said, said previous anti-cancer therapy may be selected from the group constituted of chemotherapy, targeted cancer therapies, radiotherapy, bone marrow and/or stem cell transplantation and immunotherapy.

"Chemotherapy" refers to the treatment of cancer with one or more cytotoxic antineoplastic drugs (chemotherapeutic drugs) as a part of a standardized regimen. A chemo drug is usually given in cycles, wherein a period of treatment is followed by a rest period to allow the body time to recover.

"Chemo drugs", that are used for example to treat a hematological malignancy are, without limitation to it, cytarabine (cytosine arabinoside or ara-C) and the anthracycline drugs (such as daunorubicin and/or daunomycin, doxorubicin and liposomal doxorubicin, idarubicin, and mitoxantrone), gemtuzumab, clofarabine, cladribine, hydroxyurea (hydrea®), etoposide, amsacrine, FLT3-inhibitors, and demethylating agents (5-azacytidine and decitabine), melphalan, cyclophosphamide, vincristine, proteasome inhibitors such as bortezomib, lenalidomide, thalidomide and/or pomalidomide, in particular bortezomib and/or lenalidomide.

"Targeted cancer therapies" refers to drugs or other substances that block the growth and spread of cancer by interfering with specific molecules involved in tumor growth and progression.

"Radiation therapy" or "radiation" uses high-energy radiation to remove cancer cells. Radiation therapy might be used before a bone marrow or peripheral blood stem cell transplant.

"Bone marrow and/or stem cell transplantation" refers to a cell transplantation aimed to restore stem cells that were destroyed by high doses of chemotherapy and/or radiation therapy. Sources of stem cells include bone marrow, peripheral blood or umbilical cord blood. Depending on the source of stem cells that are transplanted, the procedure might be distinguished into bone marrow transplant (BMT) or peripheral blood stem cell transplant (PBSCT) or umbilical cord blood transplantation (UCBT). Furthermore bone marrow and/or stem cell transplantation might refer to an autologous stem cell transplantation and/or an allogeneic transplantation.

In an "autologous transplant", a subject's own stem cells are removed from his or her bone marrow or peripheral blood. They are frozen and stored while the person gets treatment (high-dose chemotherapy and/or radiation). A process called "purging" may be used to try to remove any leukaemia cells in the samples. The stem cells are then reinfused into the subject's blood after treatment.

"Allogeneic transplants" are transplants from a matched donor. The advantage of allogenic bone marrow transplants is that the transplanted cells from the donor might establish a new immune system, which might detect leukaemia cells as foreign and removes them. The disadvantage of the allogeneic transplants is the limitation of matching donors and the side effects.

"Immunotherapy" refers to the stimulation of the subject's immune system to attack the malignant tumor cells that are responsible for the disease. This can be done either through immunization of the subject e.g., by administering a cancer vaccine, in which case the subject's own immune system is trained to recognize tumor cells as targets to be destroyed, or through the administration of therapeutic antibodies as drugs, in which case the subject's immune system is recruited to destroy tumor cells by the therapeutic antibodies.

In the context of the disclosure, the subject may have been previously treated for a hematological malignancy by standard anti-cancer therapy, as defined above, but relapsed and/or refracted.

Thus, in a particular embodiment, the subject suffers from CD38⁺ multiple Myeloma, in particular from relapsed and/or refractory CD38⁺ multiple Myeloma.

"Relapsed" refers to a subject in whom the hematological malignancy has been treated and improved but in whom the hematological malignancy recurred.

"Refracted" refers to a subject in whom the hematological malignancy has been treated without any improvement and the hematological malignancy thus progressed.

In one embodiment, the subject has been previously treated with bortezomib and/or lenalidomide.

In one embodiment, the subject has previously received an autologous stem cell transplant (ASCT).

In one embodiment, the subject has relapsed within 6 months after an autologous transplantation.

It is known in the art, that subjects having multiple myeloma and certain genetic features, such as the chromosomal deletion 17p, the translocations t (4, 14), t (14, 16), t (14, 20) or amplifications such as >3 copies of 1q21 are associated with a worse outcome.

Therefore in one embodiment, the subject has a 17p deletion, t (4, 14), t (14, 16), t (14, 20) and/or >3 copies of 1q21.

Recently, researchers have developed a genomic profiling test for subjects with multiple Myeloma. This test allows doctors to classify subjects with multiple myeloma based on its genomic expression profile and not just a few chromosomal abnormalities.

In one embodiment, the subject thus may have a high-risk gene expression profiling (GEP) signature (Shaughnessy JD Jr., Zhan F, Burington BE, et al. (2007), Blood 109: 2276-2284.).

The subject may represent any combination of the above mentioned features.

Multiple myeloma may be detected by the presence of monoclonal proteins (M proteins).

"M-Protein" refers to a paraprotein (a monoclonal protein, or M protein). This paraprotein is an immunoglobulin or immunoglobulin light-chain that is produced in excess by the clonal proliferation of plasma cells. Amounts higher than a certain threshold indicate multiple Myeloma. The M-protein is usually quantified in the serum as well as in the urine.

The M-protein level in the serum is measured by typically serum electrophoresis or by for example specific immunoglobulin assays; however, specific immunoglobulin quantification always overestimates the M-protein because normal immunoglobulins are included in the result. For this reason, baseline and follow-up measurements of the M-protein should be done by the same method (Riches PG et al., 1991).

In one embodiment, M-protein in serum of >0.5 g/dL indicates multiple Myeloma. In another embodiment, M-protein in serum of greater than about 0.5 g/dL indicates multiple Myeloma.

The M-protein in urine refers to the M-protein excreted in the urine measured over 24 hours. The total amount of protein excreted over 24 hours is typically measured and multiplied with the value of the percentage of urine M-protein that is determined by electrophoresis of concentrated urine protein.

In one embodiment, M-protein in urine of >200 mg in a 24-hr urine indicate multiple Myeloma. In another embodiment, M-protein in urine of greater than about 200 mg in a 24-hr urine indicates multiple Myeloma.

Multiple myeloma might be further identified by immunoglobulin light chain found in the urine, this paraprotein is called Bence Jones protein and is a urinary paraprotein composed of free light chains, wherein the light chains are lambda (λ) and/or kappa (κ) free light chains. These free light chains (FLC) may be measured by commercial tests. The free light chain measurement refers to the measurement of the FLC kappa and FLC lambda free light chains giving a free light chain ratio (FLC) of FLC kappa to FLC lambda (FLC κ/λ ratio), wherein a normal FLC κ/λ ratio ranges from 0.26 to 1.65.

In patients with multiple myeloma, either of the light chains, kappa or lambda, may be dominantly produced which results in changes of the FLC κ/λ ratio.

Abnormal FLC κ/λ ratios indicating multiple myeloma are thus FLC κ/λ ratios <0.26 or >1.65.

In one embodiment, elevated serum free light chains (FLC) with FLC >10 mg/dL and with abnormal FLC ratio indicate multiple Myeloma. In another embodiment, elevated serum free light chains (FLC) with FLC greater than about 10 mg/dL and with abnormal FLC ratio indicates multiple Myeloma.

Therefore, in one embodiment, the subject having multiple myeloma has a) measurable serum M-protein of greater than about 0.5 g/dL, and/or b) urine M-protein of greater than about 200 mg (24-hr urine), and/or c) elevated serum free light chains (FLC) with FLC greater than about 10 mg/dL with abnormal FLC ratio.

In one embodiment, the subject is tolerant to infused protein products.

The antibody in context of the disclosure binds specifically CD38. According to an embodiment, the anti-CD38 antibodies to be used in the frame of the disclosure are capable of killing a CD38⁺ cell by induction of apoptosis, antibody-dependent cell-mediated cytotoxicity (ADCC), and complement-dependent cytotoxicity (CDC).

As used herein, "apoptosis" refers to a process of programmed cell death.

"Antibody-dependent cell-mediated cytotoxicity" or "ADCC" refers to a mechanism of cell-mediated immune defense whereby an effector cell of the immune system actively lyses a target cell, whose membrane-surface antigens have been bound by specific antibodies.

"Complement-dependent cytotoxicity" or "CDC", in the context of the disclosure, refers to lysis of a target cell in the presence of complement system proteins.

As used herein, "conjugate", "immunoconjugate", "antibody-drug conjugate" or "ADC" have the same meaning and are interchangeable.

An "antibody" may be a natural or conventional antibody in which two heavy chains are linked to each other by disulfide bonds and each heavy chain is linked to a light chain by a disulfide bond. There are two types of light chain, lambda (λ) and kappa (κ). There are five main heavy chain classes (or isotypes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site.

"Complementarity Determining Regions" or "CDRs" refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

"Framework Regions" (FRs) refer to amino acid sequences interposed between CDRs, *i.e.* to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively.

As used herein, a "human framework region" is a framework region that is substantially identical (about 85%, or more, in particular 90%, 95%, 97%, 99% or 100%) to the framework region of a naturally occurring human antibody.

CDR/FR definition concerning the immunoglobulin light or heavy chain are given based on the Kabat definition (http://www.bioinf.org.uk/abs/).

As used herein, the term "antibody" denotes conventional antibodies and fragments thereof, and chimeric, humanized, bispecific or multispecific antibodies.

The term "monoclonal antibody" or "mAb" as used herein refers to an antibody molecule of a single amino acid composition that is directed against a specific antigen, and is not to be construed as requiring production of the antibody by any particular method. A monoclonal antibody may be produced by a single clone of B cells or hybridoma, but may also be recombinant, *i.e.* produced by protein engineering.

The term "chimeric antibody" refers to an engineered antibody in which the constant region or a portion thereof, is altered, replaced, or exchanged, so that the variable region is linked to a constant region of a different species, or belonging to another antibody class or subclass. A chimeric antibody also refers to an antibody in which the variable region or a portion thereof, is altered, replaced, or exchanged, so that the constant region is linked to a variable region of a different species, or belonging to another antibody class or subclass. In particular a chimeric antibody comprises a VH domain and a VL domain of an antibody derived from a non-human animal, in association with a CH domain and a CL domain of another antibody, in particular a human antibody. As the non-human animal, any animal such as mouse, rat, hamster, rabbit or the like can be used. A chimeric antibody may also denote a multispecific antibody having specificity for at least two different antigens. In an embodiment, a chimeric antibody has variable domains of mouse origin and constant domains of human origin.

The term "humanized antibody" refers to an antibody which is initially wholly or partially of non-human origin and which has been modified to replace certain amino acids, in particular in the framework regions of the heavy and light chains, in order to avoid or minimize an immune response in humans. The constant domains of a humanized antibody are most of the time human CH and CL domains. In an embodiment, a humanized antibody has constant domains of human origin.

For chimeric antibodies, humanization typically involves modification of the framework regions of the variable region sequences.

Amino acid residues that are part of a CDR will typically not be altered in connection with humanization, although in certain cases it may be desirable to alter individual CDR amino acid residues, for example to remove a glycosylation site, a deamidation site, an undesired cysteine residue, a lysine residue in the case of ADC. N-linked glycosylation occurs by attachment of an oligosaccharide chain to an asparagine residue in the tripeptide sequence Asn-X-Ser or Asn-X-Thr, where X may be any amino acid except Pro. Removal of an N-glycosylation site may be achieved by mutating either the Asn or the Ser and/or Thr residue to a different residue, in particular by way of conservative amino acid substitution. Deamidation of asparagine and glutamine residues can occur depending on factors such as pH and surface exposure. Asparagine residues are particularly susceptible to deamidation, primarily when present in the sequence Asn-Gly, and to a lesser extent in other dipeptide sequences such as Asn-Ala. When such a deamidation site, in particular Asn-Gly, is present in a CDR sequence, it may therefore be desirable to remove the site, typically by conservative substitution to remove one of the implicated residues. In the case of ADC, attachment of a cytotoxic to mAb could be prepared via covalent linkage to lysine side chain residue. This steric hindrance may interfere with mAb binding to antigen. It may therefore be desirable to remove the lysine residue, typically by an arginine conservative substitution. Substitution in a CDR sequence to remove one of the implicated residues is also intended to be encompassed by the present disclosure.

The goal of humanization is a reduction in the immunogenicity of a xenogenic antibody, such as a murine antibody, for introduction into a human, while maintaining the full antigen binding affinity and specificity of the antibody. Humanized antibodies, or antibodies adapted for non-rejection by other mammals, may be produced using several technologies such as resurfacing and CDR grafting. As used herein, the resurfacing technology uses a combination of molecular modeling, statistical analysis and mutagenesis to alter the non-CDR surfaces of antibody variable regions to resemble the surfaces of known antibodies of the target host.

Strategies and methods for the resurfacing of antibodies, and other methods for reducing immunogenicity of antibodies within a different host, are disclosed in U.S. Patent No. 5,639,641. Briefly, in a preferred method, (1) position alignments of a pool of antibody heavy and light chain variable regions is generated to give a set of heavy and light chain variable region framework surface exposed positions wherein the alignment positions for all variable regions are at least about 98% identical; (2) a set of heavy and light chain variable region framework surface exposed amino acid residues is defined for a rodent antibody (or fragment thereof); (3) a set of heavy and light chain variable region framework surface exposed amino acid residues that is most closely identical to the set of rodent surface exposed amino acid residues is identified; (4) the set of heavy and light chain variable region framework surface exposed amino acid residues defined in step (2) is substituted with the set of heavy and light chain variable region framework surface exposed amino acid residues identified in step (3), except for those amino acid residues that are within 5 Å of any atom of any residue of the complementarity-determining regions of the rodent antibody; and (5) the humanized rodent antibody having binding specificity is produced. Thus in one embodiment humanized antibodies may also be called "resurfaced" antibodies.

Antibodies can be further humanized using a variety of other techniques including CDR-grafting (EP0239400; WO91/09967; U.S. Patent Nos. 5,530,101 and 5,585,089), veneering or resurfacing (EP0592106; EP0519596; Padlan (1991) Molecular Immunology 28(4/5):489-498; Studnicka et al. (1994) Protein Engineering 7(6):805-814; Roguska et al. (1994) Proc. Natl. Acad. Sci U.S.A. 91:969-973), and chain shuffling (U.S. Patent No. 5,565,332). Human antibodies can be made by a variety of methods known in the art including phage display methods. See also U.S. Patent Nos. 4,444,887, 4,716,111, 5,545,806, and 5,814,318; and International patent application WO98/46645, WO98/50433, WO98/24893, WO98/16654, WO96/34096, WO96/33735, and WO91/10741.

A "conservative amino acid substitution" is one in which an amino acid residue is substituted by another amino acid residue having a side chain R group with similar chemical properties (e.g., charge or hydrophobicity). In general, a conservative amino acid substitution will not substantially change the functional properties of a protein. Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups are: valine-leucine-isoleucine, phenylalanine-tyrosine-tryptophane, lysine-arginine, alanine-valine, glutamate-aspartate, and asparagineglutamine.

"Fragments" of (conventional) antibodies comprise a portion of an intact antibody, in particular the antigen binding region or variable region of the intact antibody. Examples of antibody fragments include Fv, Fab, F(ab')₂, Fab', dsFv, (dsFv)₂, scFv, sc(Fv)₂, diabodies, bispecific and multispecific antibodies formed from antibody fragments.

The term "Fab" denotes an antibody fragment having a molecular weight of about 50,000 Da and antigen binding activity, in which about a half of the N-terminal side of H chain and the entire L chain, among fragments obtained by treating IgG with a protease, papaine, are bound together through a disulfide bond.

The term "F(ab')₂" refers to an antibody fragment having a molecular weight of about 100,000 Da and antigen binding activity, which is slightly larger than the Fab bound via a disulfide bond of the hinge region, among fragments obtained by treating IgG with a protease, pepsin.

The term "Fab"' refers to an antibody fragment having a molecular weight of about 50,000 Da and antigen binding activity, which is obtained by cutting a disulfide bond of the hinge region of the F(ab')₂ fragment.

A single chain Fv ("scFv") polypeptide is a covalently linked VH::VL heterodimer which is usually expressed from a gene fusion including VH and VL encoding genes linked by a peptide-encoding linker. The human scFv fragment of the disclosure includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)₂.

"dsFv" is a VH::VL heterodimer stabilised by a disulphide bond.

"(dsFv)₂" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term "multispecific antibody" denotes an antibody which combines the antigen-binding sites of two or more antibodies within a single molecule.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

The antibody to be used in the in frame of the disclosure may be one of the anti-CD38 monoclonal antibodies referred to as 38SB13, 38SB18, 38SB19, 38SB30, 38SB31 and 38SB39, which are described in WO2008/047242, or derivatives thereof obtained through the resurfacing technology.

In particular, the anti-CD38 antibody in context of the disclosure comprises at least one heavy chain comprising CDR-H1 of sequence SEQ ID NO: 1, CDR-H2 of sequence SEQ ID NO: 2, and CDR-H3 of sequence SEQ ID NO: 3, and at least one light chain comprising three sequential CDRs having amino acid sequences consisting of SEQ ID NOS: 4, 5, and 6. In particular, said antibody has a heavy chain variable domain comprising SEQ ID NO: 44 and a light chain variable domain comprising SEQ ID NO: 38 chain.

In particular, the anti-CD38 antibody in context of the disclosure comprises at least one heavy chain comprising CDR-H1 of sequence SEQ ID NO: 7, CDR-H2 of sequence SEQ ID NO: 8, and CDR-H3 of sequence SEQ ID NO: 9, and at least one light chain comprising three sequential CDRs having amino acid sequences consisting of SEQ ID NOS: 10, 11, and 12. In particular, said antibody has a heavy chain variable domain comprising SEQ ID NO: 45 and a light chain variable domain comprising SEQ ID NO: 39 chain.

In particular, the anti-CD38 antibody in context of the disclosure comprises at least one heavy chain comprising CDR-H1 of sequence SEQ ID NO: 13, CDR-H2 of sequence SEQ ID NO: 14, and CDR-H3 of sequence SEQ ID NO: 15, and at least one light chain comprising three sequential CDRs having amino acid sequences consisting of SEQ ID NOS: 16, 17, and 18. In particular, said antibody has a heavy chain variable domain comprising SEQ ID NO: 46 and a light chain variable domain comprising SEQ ID NO: 40 chain.

In particular, the anti-CD38 antibody in context of the disclosure comprises at least one heavy chain comprising CDR-H1 of sequence SEQ ID NO: 19, CDR-H2 of sequence SEQ ID NO: 20, and CDR-H3 of sequence SEQ ID NO: 21, and at least one light chain comprising three sequential CDRs having amino acid sequences consisting of SEQ ID NOS: 22, 23, and 24. In particular, said antibody has a heavy chain variable domain comprising SEQ ID NO: 47 and a light chain variable domain comprising SEQ ID NO: 41 chain.

In particular, the anti-CD38 antibody in context of the disclosure comprises at least one heavy chain comprising CDR-H1 of sequence SEQ ID NO: 25, CDR-H2 of sequence SEQ ID NO: 26, and CDR-H3 of sequence SEQ ID NO: 27, and at least one light chain comprising three sequential CDRs having amino acid sequences consisting of SEQ ID NOS: 28, 29, and 30. In particular, said antibody has a heavy chain variable domain comprising SEQ ID NO: 48 and a light chain variable domain comprising SEQ ID NO: 42 chain.

In particular, the anti-CD38 antibody in context of the disclosure comprises at least one heavy chain comprising CDR-H1 of sequence SEQ ID NO: 31, CDR-H2 of sequence SEQ ID NO: 32, and CDR-H3 of sequence SEQ ID NO: 33, and at least one light chain comprising three sequential CDRs having amino acid sequences consisting of SEQ ID NOS: 34, 35, and 36. In particular, said antibody has a heavy chain variable domain comprising SEQ ID NO: 49 and a light chain variable domain comprising SEQ ID NO: 43 chain.

Further enclosed in the context of the disclosure are antibodies comprising a sequence with at least 85%, more particularly at least 90%, 91%, 92%, 93%, 94%, 95%, 96%, 97%, 98%, 99% or 100% identical to the sequenced herein disclosed.

A sequence "at least 85% identical to a reference sequence" is a sequence having, on its entire length, 85%, or more, in particular 90%, 91%, 92%, 93%, 94%, 95%, 96%, 97%, 98% or 99% sequence identity with the entire length of the reference sequence.

A percentage of "sequence identity" may be determined by comparing the two sequences, optimally aligned over a comparison window, wherein the portion of the polynucleotide or polypeptide sequence in the comparison window may comprise additions or deletions (*i.e.* gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison is conducted by global pairwise alignment, e.g. using the algorithm of Needleman and Wunsch J. Mol. Biol. 48: 443 (1970). The percentage of sequence identity can be readily determined for instance using the program Needle, with the BLOSUM62 matrix, and the following parameters gap-open=10, gap-extend=0.5.

In one embodiment, the anti-CD38 antibodies are humanized anti-CD38 antibodies obtained through the resurfacing technology. Such antibodies may also be called "resurfaced" antibodies.

In one aspect, a resurfaced and/or humanized version of the antibody comprises at least one heavy chain and at least one light chain, wherein said heavy chain comprises three sequential complementarity-determining regions having amino acid sequences represented by SEQ ID NOS: 1, 2, and 3, and wherein said light chain comprises three sequential complementarity-determining regions having amino acid sequences represented by SEQ ID NOS: 4, 5, and 6.

In another aspect, a resurfaced and/or humanized version of the antibody comprises at least one heavy chain and at least one light chain, wherein said heavy chain comprises three sequential complementarity-determining regions having amino acid sequences represented by SEQ ID NOS: 7, 8, and 9, and wherein said light chain comprises three sequential complementarity-determining regions having amino acid sequences represented by SEQ ID NOS: 10, 11, and 12.

In another embodiment, a resurfaced and/or humanized version of the antibody comprises at least one heavy chain and at least one light chain, wherein said heavy chain comprises three sequential complementarity-determining regions having amino acid sequences represented by SEQ ID NOS: 13, 37, and 15, and wherein said light chain comprises three sequential complementarity-determining regions having amino acid sequences represented by SEQ ID NOS: 16, 17, and 18.

In another aspect, a resurfaced and/or humanized version of the antibody comprises at least one heavy chain and at least one light chain, wherein said heavy chain comprises three sequential complementarity-determining regions having amino acid sequences represented by SEQ ID NOS: 19, 20, and 21, and wherein said light chain comprises three sequential complementarity-determining regions having amino acid sequences represented by SEQ ID NOS: 22, 23, and 24.

In another aspect, a resurfaced and/or humanized version of the antibody comprises at least one heavy chain and at least one light chain, wherein said heavy chain comprises three sequential complementarity-determining regions having amino acid sequences represented by SEQ ID NOS: 25, 26, and 27, and wherein said light chain comprises three sequential complementarity-determining regions having amino acid sequences represented by SEQ ID NOS: 28, 29, and 30.

In a further aspect, a resurfaced and/or humanized version of the antibody comprises at least one heavy chain and at least one light chain, wherein said heavy chain comprises three sequential complementarity-determining regions having amino acid sequences represented by SEQ ID NOS: 31, 32, and 33, and wherein said light chain comprises three sequential complementarity-determining regions having amino acid sequences represented by SEQ ID NOS: 34, 35, and 36.

In one aspect, this disclosure provides resurfaced and/or humanized antibodies which comprise a V_{H} having an amino acid sequence selected from the group of SEQ ID NOS: 50 and 51. In a further embodiment, a resurfaced and/or humanized version of the antibody comprises a V_{H} having an amino acid sequence represented by SEQ ID NO: 50. In another aspect, a humanized version of the antibody comprises a V_{H} having an amino acid sequence represented by SEQ ID NO: 51.

In another aspect, this disclosure provides humanized antibodies which comprise a V_{L} having an amino acid sequence selected from the group of SEQ ID NOS: 52, 53, 54, and 55. In another aspect, a humanized version of the antibody comprises a V_{L} having an amino acid sequence chosen from the group of SEQ ID NOS: 52 and 53. In another aspect, a humanized version of the antibody comprises a V_{L} having an amino acid sequence chosen from the group of SEQ ID NOS: 54 and 55.

In a specific embodiment, the antibody according to the disclosure is the humanized and/or resurfaced antibody referred to as hu38SB19, which comprises a V_{H} having an amino acid sequence represented by SEQ ID NO: 50 and a comprises a V_{L} having an amino acid sequence represented by SEQ ID NO: 52.

In another embodiment, the antibody according to the disclosure is daratumubab.

In a specific embodiment, the antibody for use according to the disclosure is a naked antibody, i.e., it is not linked to any drug in order to form an antibody-drug conjugate.

The antibodies for use according to the disclosure specifically bind CD38 and are capable of killing a CD38⁺ cell by induction of apoptosis, antibody-dependent cell-mediated cytotoxicity (ADCC). In a specific embodiment, the antibodies for use according to the disclosure are capable of killing a CD38⁺ cell *in vitro* by induction of apoptosis even when the cells treated with the antibody have not been cross-linked with anti-human IgG secondary antibody.

Further enclosed, in the context of the disclosure, are humanized antibodies comprising a sequence with at least 85%, more particularly at least 90%, 91%, 92%, 93%, 94%, 95%, 96%, 97%, 98%, 99% or 100% identical to the sequences disclosed herein above.

The antibodies according to the disclosure may be obtained by standard techniques of animal immunization, hybridoma formation and selection for antibodies with specific characteristics. In particular, the CD38 antibody 38SB19 is obtained as described in example 4 of the international patent application WO2008/047242, i.e. according to the following protocol of cloning and sequencing of the light and heavy chains of anti-CD38 antibodies :
(i) RNA preparation from hybridoma cells that produces the 38SB19 antibody as follow: preparations of total RNA were obtained from 5 x 10⁶ hybridoma cells, which produce 38SB19 antibody, using Qiagen's RNeasy miniprep kit. Briefly, 5 x 10⁶ cells were pelleted and resuspended in 350 µL RLT buffer (containing 1% β-mercaptoethanol). The suspension was homogenized by passing it through a 21.5 gauge needle and syringe roughly 10 - 20 times or until it was no longer viscous. Ethanol (350 µL of 70% aqueous ethanol) was added to the homogenate, which was mixed well. The solution was transferred to a spin column, placed in a 2-mL collection tube and spun at >8000 x g for 15 seconds. The column was washed twice with 500 µL RPE buffer, then transferred to a fresh tube and eluted with 30 µL RNase free water and a 1-minute spin. The eluate (30 µL) was placed back on the column for a second 1-minute elution spin. An aliquot of the 30 µL eluate was diluted with water and used to measure the UV absorption at 260 nm for RNA quantitation.
(ii) cDNA Preparation with Reverse Transcriptase (RT) reaction as follow : the variable region 38SB19 antibody cDNA was generated from the total RNA using Invitrogen's Superscriptll kit. The kit protocols were followed closely, utilizing up to 5 µg of total RNA from the Qianeasy mini preps. Briefly, the RNA, 1 µL random primers, and 1 µL dNTP mix were brought up to 12 µL with RNase free sterile distilled water and incubated at 65°C for 5 minutes. The mix was then put on ice for at least 1 minute. Next 4 µL of 5 x reaction buffer, 2 µL 0.1 M DTT, and 1 µL RNaseOUT were added and the mix was incubated at 25°C for 2 minutes in an MJ Research thermalcycler. The thermalcylcer was paused so that 1 µL of Superscriptll enzyme could be added and then restarted for an additional 10 minutes at 25°C before shifting to 55°C for 50 minutes. The reaction was heat inactivated by heating to 70°C for 15 min and the RNA was removed by adding 1 µL RNase H and incubating at 37°C for 20 minutes.
(iii) Degenerate PCR reactions : the procedure for the first round degenerate PCR reaction on the cDNA derived from hybridoma cells was based on methods described in Wang et al. (2000) and Co et al.(1992). The primers for this round (Table 1) contain restriction sites to facilitate cloning into the pBluescriptll plasmids.

**Table 1 : Primers used for the degenerate PCR reactions**

| Primer | Sequence |
|---|---|
| BamIgG1 (SEQ ID NO. 56) | |
| IgG2Abam (SEQ ID NO. 57) | GGAGGATCCCTTGACCAGGCATCCTAGAGTCA |
| EcoMH 1 (SEQ ID NO. 58) | CTTCCGGAATTCSARGTNMAGCTGSAGSAGTC |
| EcoMH2 (SEQ ID NO. 59) | |
| SaclMK (SEQ ID NO. 60) | GGAGCTCGAYATTGTGMTSACMCARWCTMCA |
| HindKL (SEQ ID NO. 61) | |

Primers used for the degenerate PCR reactions are based on those in Wang *et al.,* 2000 except HindKL which is based on Co *et al.* 1992. Mixed bases are defined as follows: H=A+T+C, S=G+C, Y=C+T, K= G+T, M=A+C, R=A+G, W=A+T, V = A+C+G.
The PCR reaction components (Table 2) were mixed on ice in thin walled PCR tubes and then transferred to an MJ research thermalcycler preheated and paused at 94°C. The reactions were performed using a program derived from Wang et al., 2000 as follows:
Name: Wang45
94°C 3:00 min
94°C 0:15 sec
45°C 1:00 min
72°C 2:00 min
Goto 2 29 times
72°C 6:00 min
4°C for ever
End.

The PCR reaction mixtures were then run on a 1% low melt agarose gel, the 300 to 400 bp bands were excised, purified using Zymo DNA mini columns, and sent to Agencourt biosciences for sequencing. The respective 5' and 3' PCR primers were used as sequencing primers to generate the 38SB19 variable region cDNAs from both directions.
(iv) Cloning the 5' end sequence as follow : Since the degenerate primers used to clone the 38SB19 variable region light chain and heavy chain cDNA sequences alters the 5' end sequences, additional sequencing efforts were needed to decipher the complete sequences. The preliminary cDNA sequence from the methods described above were used to search the NCBI IgBlast site (http://www.ncbi.nlm.nih.gov/igblast/) for the murine germline sequences from which the 38SB19 sequence is derived. PCR primers were designed (Table 3) to anneal to the leader sequence of the murine antibody so that a new PCR reaction could yield the complete variable region cDNA, unaltered by the PCR primers. The PCR reactions, band purifications, and sequencing were performed as described above.

**Table 2 : The light and heavy chain PCR reaction mixes for cloning of the 38SB19 variable region cDNA sequences.**

| Light Chain Reaction Mix | Heavy Chain Reaction Mix |
|---|---|
| 5 µl 10 X PCR reaction buffer (Roche) | 5 µl 10 X PCR reaction buffer (Roche) |
| 4 µl 10mM dNTP mix (2.5mM each) | 4 µl 10mM dNTP mix (2.5mM each) |
| 2 µl Template (RT reaction) | 2 µl Template (RT reaction) |
| 5 µl 10 µM Sac1 MK left primer | 2.5 µl 10 µM EcoMH1 left primer |
| 5 µl 10 µM HindKL right primer | 2.5 µl 10 µM EcoMH2 left primer |
| | 5 µl 10 µM BamIgG1 right primer |
| 5 µl DMSO | 5 µl DMSO |
| 0.5 µl Taq Polymerase (Roche) | 0.5 µl Taq Polymerase (Roche) |
| 23.5 µl sterile distilled H₂O | 23.5 µl sterile distilled H₂O |
| 50 µl Total | 50 µl Total |

(v) Peptide analysis for sequence confirmation as follow: The cDNA sequence information for the variable region was combined with the germline constant region sequence to obtain full length antibody cDNA sequences. The molecular weights of the heavy chain and light chain were then calculated and compared with the molecular weights obtained by LC/MS analyses of the murine 38SB19 antibody. Table 5 of U.S. Patent 8,153,765 gives the calculated mass from the cDNA sequences for 38SB19 LC and HC together with the values measured by LC/MS. The molecular weight measurements are consistent with the cDNA sequences for both the 38SB19 light and heavy chain.

**Table 3 : The 5' end murine leader sequence primers used for the 38SB19 second round PCR reactions.**

| Primer | Sequence |
|---|---|
| Light Chain | |
| 38SB19 LC Leader (SEQ ID NO. 62) | ATGGAGTCACAGATTCAGGTC |
| Heavy Chain 38-19HCLead1 (SEQ ID NO. 63) | TTTTGAATTCCAGTAACTTCAGGTGTCCACTC |

Table 3 provides the 5' end murine leader sequence primers used for the 38SB19 second round PCR reactions. The 3' end primers are identical to those used in the first round reactions since they prime to the respective constant region sequences.

More particularly, the humanized 38SB19 antibodies may be produced as described in example 5 of the international patent application WO2008/047242, i.e. according to the following protocol : The variable region sequences for hu38SB19 were codon-optimized and synthesized by Blue Heron Biotechnology. The sequences are flanked by restriction enzyme sites for cloning in-frame with the respective constant sequences in both single chain and the tandem dual chain mammalian expression plasmids. The light chain variable region is cloned into EcoRI and BsiWI sites in both the ps38SB19LCZv1.0 and ps38SB19v1.00 plasmids (FIG. 2A and 2C of WO2008/047242). The heavy chain variable region is cloned into the Hindlll and Apa1 sites in both the ps38SB19HCNv1.0 and ps38SB19v1.00 plasmids (FIG. 2B and 2C of WO2008/047242). These plasmids can be used to express hu38SB19 in either transient or stable transfections in mammalian cells. Similar expression vector constructs were used to produce other chimeric and humanized antibodies. Transient transfections to express hu38SB19 in HEK-293T cells were performed using CaPO₄ reagents from BD biosciences. The supplied protocols were slightly modified for enhanced expression yields. Briefly, 2 x 10⁶ HEK-293T cells were plated on 10 cm tissue culture plates coated with polyethyleneimine (PEI) 24 h prior to transfection. The transfection began by washing the cells with PBS and replacing the media with 10 mL DMEM (Invitrogen) with 1% Ultra Low IgG FBS (Hyclone). Solution A (10 µg DNA, 86.8 µL Ca²⁺ solution, and up to 500 µL with H₂O) was added drop wise to Solution B while vortexing. The mixture was incubated at RT for 1 min and 1 mL of the mixture was added drop wise to each 10 cm plate. Approximately 16 h post transfection, media was replaced with 10 mL fresh DMEM with 1% Ultra Low IgG FBS. Approximately 24 hours later 2 mM sodium butyrate was added to each 10 cm plate. The transfection was harvested 4 days later. Supernatant was prepared for Protein A affinity chromatography by the addition of 1/10 volume of 1 M Tris/HCI buffer, pH 8.0. The pH-adjusted supernatant was filtered through a 0.22 µm filter membrane and loaded onto a Protein A Sepharose column (HiTrap Protein A HP, 1 mL, Amersham Biosciences) equilibrated with binding buffer (PBS, pH 7.3). A Q-Sepharose precolumn (10 mL) was connected upstream of the Protein A column during sample loading to reduce contamination from cellular material such as DNA. Following sample loading, the precolumn was removed and the Protein A column orientation was reversed for wash and elution. The column was washed with binding buffer until a stable baseline was obtained with no absorbance at 280 nm. Antibody was eluted with 0.1 M acetic acid buffer containing 0.15 M NaCl, pH 2.8, using a flow rate of 0.5 mL/min. Fractions of approximately 0.25 mL were collected and neutralized by the addition of 1/10 volume of 1M Tris/HCl, pH 8.0. The peak fraction(s) was dialysed overnight twice against PBS and purified antibody was quantitated by absorbance at OD₂₈₀. Humanized and chimeric antibodies can also be purified using a Protein G column with slightly different procedures.

All the exemplified chimeric, humanized and/or resurfaced anti-CD38 antibodies were expressed and purified using procedures similar to those described above.

The inventors determined for the antibodies of the disclosure the suitable dose of administration and regimen in order to obtain a well-tolerated anti-cancer treatment that enables treating subjects suffering from a CD38⁺ hematological malignancy, in particular, from CD38⁺ multiple Myeloma, more particularly from relapsed and/or refractory CD38⁺ multiple Myeloma.

Therefore, an accelerated dose escalation schedule was used for the first five dose levels ranging from 0.0001 mg/kg to 0.1 mg/kg, administered every two weeks, with one evaluable subject per dose level. All subsequent dose levels such as 0.3 mg/kg, 1 mg/kg, 3 mg/kg, 5mg/kg, 10 mg/kg and 20 mg/kg were administered every 2 weeks, 10 mg/kg was also administered every week, followed the classic 3+3 design for dose escalation based on dose level toxicity, the median number of cycles ranged from 2.0 to 50.0 for the administration.

Within this study, the inventors demonstrated that the antibody used has a safety profile that is manageable with a maximum tolerated dose that is higher than 10 mg/kg.

Immunogenicity studies performed by the inventors showed that the human subject did not produce antibodies against anti-CD38 antibody.

Furthermore, the inventors could show that the antibody of the disclosure has in humans high binding efficiency, demonstrated by the high receptor occupancy at low doses. Receptor occupancy was detected for example at a dose level of 1 mg/kg and reached a range of 84.1 to 97.7 % at 10 mg/kg every two weeks.

Receptor occupancy is typically assessed using two monoclonal antibodies (mAbs) binding to two different epitopes of the CD38 antigen. MAb1 is specific for the same epitope as e.g. hu38SB19, and thus a reporter of the free CD38 sites (unoccupied by e.g. hu38SB19). The second monoclonal antibody, mAb2, directed to a different epitope than that of hu38SB19, provides a positive control for the presence of CD38⁺ cells and an evaluation of the amount of CD38 antigen remaining on the cell surface after *in vitro* drug spiking. The use of beads as calibrators and an indirect detection allows a quantitative approach without any modification of binding capacity of hu38SB19. The combination of the results provides the receptor density and occupancy per cell.

There was no significant increase of Cₘₐₓ at the cycle 2 compared to Cₘₐₓ at cycle 1 for the 0.03 to 3 mg/kg dose range.

The inventors further demonstrate that the antibody reached tumor growth inhibition with a threshold at Cₘₐₓ for 1 patient at dose level of 5 mg/kg and 5 patients at a dose level of 10 mg/kg.

The inventors have demonstrated in particular that a subject suffering from a CD38⁺ hematological malignancy, in particular from CD38⁺ multiple Myeloma, most particular relapsed and/or refractory CD38⁺ multiple Myeloma, showed a particular response when the anti-CD38 antibody of the disclosure was administrated at a dose of 1 mg/kg, 5 mg/kg and 10 mg/kg.

The inventors observed, in particular, that a tumor growth inhibition threshold was reached at Cₘₐₓ for 1 patient at a dose level of 5 mg/kg and for 5 patients at a dose level of 10 mg/kg.

According to the disclosure, the antibody is for use as a medicament, wherein said antibody is administered to a human subject in a safe therapeutic dose of 20 mg/kg or below.

In one embodiment, the antibody is for use for treating a CD38⁺ hematological malignancy, in particular for treating multiple Myeloma, most particular for treating relapsed and/or refractory multiple Myeloma.

CD38⁺ hematological malignancies to be treated in the context of the disclosure are defined in the section *"CD38⁺ hematological malignancies".*

The human subject is defined above in the section *"subject".*

In the context of the disclosure, the term "treating" or "treatment", as used herein, means reversing, alleviating, inhibiting the progress of, or preventing the disorder or condition to which such term applies, or one or more symptoms of such disorder or condition.

By the term "treating a CD38⁺ hematological malignancy" as used herein is meant the inhibition of the growth of CD38⁺ malignant cells of a tumour and/or the progression of metastases from said CD38⁺ tumor. Such treatment can also lead to the regression of tumor growth, *i.e.,* the decrease in size of a measurable tumor. In particular, such treatment leads to the complete regression of the CD38⁺ tumor or CD38⁺ metastases.

By a "therapeutically effective amount" of the antibody, in context of the disclosure, is meant a sufficient amount of the antibody to treat said CD38⁺ hematological malignancy as discovered by the inventors and disclosed herein.

In a particular aspect, said therapeutically effective amount of the antibody administered to the subject is a dose ranging from 0.0001 mg/kg to 20 mg/kg, in particular a dose ranging from 1 mg/kg to 20 mg/kg, more particularly 0.1 mg/kg, 1 mg/kg, 2 mg/kg, 3 mg/kg, 4 mg/kg, 5 mg/kg, 6 mg/kg, 7 mg/kg, 7.5 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg or 20 mg/kg.

In one aspect, the antibody of the disclosure may be administered once a week or once every two week.

In another aspect, said therapeutically effective amount of the antibody administered to the subject is a dose ranging from 1 mg/kg to 20 mg/kg, 3 mg/kg to 20 mg/kg, 5 mg/kg to 20 mg/kg, or 10 mg/kg to 20 mg/kg, for example once a week or once in two weeks. Indeed, these doses have been shown to be safe while an efficacy has been shown in some individuals.

In still another aspect, said therapeutically effective amount of the antibody administered to the subject is a dose of 10 mg/kg once a week, or of 20 mg/kg, for example once a week or once in two weeks.

In some aspects, the antibody of the disclosure may be administered according to an intermittent program with an interval between each administration of 1 week or 2 weeks, which may be prolonged by 1 to 2 weeks depending on the tolerance to the preceding administration. However, the inventors observed mostly side effects that were not severe.

Accordingly, in a particular embodiment, the administration of the antibody is repeated as a new cycle directly after the previous cycle.

A "cycle" as used herein refers in case of a 'once a week' administration to one week, in case of a 'once in two weeks' administration one cycle corresponds to two weeks.

In one embodiment the number of cycles of the administration may be 2 to 50, in particular 2, 3, 4, 5, 6, 7, 8, 9, 0, 12, 14, 16, 18, 20, 25, 30, 35, 45, 50 cycles.

The number of cycles of the administration of the antibody of the disclosure may thus be selected from the group constituted of 2, 3, 4, 5, 6, 7, 8, 9, 0, 12, 14, 16, 18, 20, 25, 30, 35, 45, 50 cycles.

The antibody of the disclosure is administered intravenously.

In some embodiments, the dose might be increased during treatment after a disease response evaluation.

The minimal dose in context of the disclosure corresponds to 0.0001 mg/kg, wherein a dose level of 0.0001 mg/kg represent 10% of theoretical CD38 receptor occupancy on normal B and T cells.

In some embodiments, the intravenous administration takes place at a certain rate of infusion.

In particular, the antibody will be administered at an initial rate of infusion of 0.042 mg/hr to 250 mg/hr, in particular the initial rate of infusion is 0.042 mg/hr,

In some embodiments, the initial rate of infusion depends on the dose to be administered.

Accordingly, in one example, the antibody of the disclosure is typically administered for example for a dose of 0.0001 mg/kg at an initial rate of 0.042 mg/hr for a total of 3 ml, for example for a dose of 0.001 mg/kg at an initial rate of 0.42 mg/hr for a total of 3 ml, for example for a dose of 0.01 mg/kg at an initial rate of 1. 4 mg/hr, for example for a dose of 0.03 mg/kg at an initial rate of 4.2 mg/hr, example for a dose of 0.1 mg/kg at an initial rate of 7 mg/hr, example for a dose of 0.01 mg/kg at an initial rate of 1.4 mg/hr, for example for a dose of 0.3 mg/kg at an initial rate of 10.5 mg/hr, for example for a dose of 1 mg/kg at an initial rate of 17.5 mg/hr, for example for a dose of 3 mg/kg at an initial rate of 52.5 mg/hr, for example for a dose of 5 mg/kg at an initial rate of 87.5 mg/hr, for example for a dose of 10 mg/kg at an initial rate of 175 mg/hr and for example for a dose of 20 mg/kg at an initial rate of 250 mg/hr.

During the administration the subject is typically observed for signs of hypersensitivity reactions, and the administration is only continued in the absence of hypersensitivity reactions.

However, it will be understood, that exact time of administration (once a week or once every two weeks), the number of cycles and the initial rate of infusion will be within the range of values disclosed herein but, however, the exact values will be decided by the attending physician for any particular subject depending upon factors including the CD38⁺ hematological malignancy being treated and the severity of the disorder; activity of the specific antibody employed; the specific composition employed, the age, body weight, general health, sex and diet of the subject.

It will be understood that the attending physician might modify and adapt the administration regime based on the disease response.

"Disease response" may be determined according to standard criteria for hematological malignancies and staging.

Methods to evaluate the disease response of a hematological malignancy, in particular a CD38⁺ hematological malignancy are known to the skilled in the art.

Typically the methods to evaluate the disease response may be selected from the group constituted of Karnofsky performance status evaluation, quantification of specific markers, bone marrow biopsy and/or aspiration, radiologic imaging of plasmocytoma, Bone skeletal survey, M-protein quantification (serum and/or 24-hr urine) and serum free light chain levels or urinary light chain levels, serum β2-microglobulin, Lymph node biopsy and/or radiologic tumor assessment (by X-ray, computed tomography [CT] scan, PET scan, or magnetic resonance imaging [MRI]), Blood count including blast count.

The best methods to evaluate the disease response of a hematological malignancy, depends on the type of CD38⁺ hematological malignancy and are known to the skilled in the art.

Based on the results obtained from the evaluation of the disease response the disease response may then be stratified according to the standard criteria for underlining disease and classified into complete response or complete remission (CR), partial response (PR), stable disease (SD) or progressive disease (PD).

The "Karnofsky score" refers to a score from 100 to 0, wherein 100 is "perfect" health and 0 is death.

"Markers" used in the context of the response evaluation may include typically serum and/or plasma markers, without limitation to them, such as Hs-CRP, Tumor necrosis factor alpha (TNF-α), IL-6, IL-1-β, IFN-λ and the CD38 receptor density and occupancy.

In one example techniques to evaluate the disease response in a subject suffering from multiple myeloma are bone marrow biopsy and/or aspiration, radiologic imaging of plasmocytoma, Bone skeleton survey, M-protein quantification as explained above, and measure of serum β2-microglobulin.

The disease response evaluation may further include receptor density and receptor occupancy on circulating tumor cells (peripheral blood), receptor density and receptor occupancy on blasts and plasma cells in bone marrow and level of human anti-drug antibodies (ADA).

The antibody of the disclosure may be administered in the form of a pharmaceutical composition including pharmaceutically acceptable excipient, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions including the antibody of the disclosure naturally depend upon the condition to be treated, the severity of the illness, the age, weight, and gender of the subject, etc.

The antibody of the disclosure is formulated for intravenous administration.

In particular, the pharmaceutical compositions including the antibody of the disclosure may contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

To prepare pharmaceutical compositions, an effective amount of the antibody of the disclosure may be dissolved or dispersed in a pharmaceutically acceptable carrier or aqueous medium.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The carrier can be a solvent or dispersion medium containing, for example, water, ethanol, polyol (for example, glycerol, propylene glycol, and liquid polyethylene glycol, and the like) and suitable mixtures thereof. The proper fluidity can be maintained, for example, by the use of a coating, such as lecithin, by the maintenance of the required particle size in the case of dispersion and by the use of surfactants, stabilizing agents, cryoprotectants or antioxidants. The prevention of the action of microorganisms can be brought about by antibacterial and antifungal agents. In many cases, it will be preferable to include isotonic agents, for example, sugars or sodium chloride.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For intravenous administration in an aqueous solution, for example, the solution should be suitably buffered if necessary and the liquid diluent first rendered isotonic with sufficient saline or glucose. These particular aqueous solutions are especially suitable for intravenous administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected intravenous at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

In one example the antibody is formulated for intravenous administration, therefore the antibody is presented as a concentrate for solution for infusion in vials, e.g. containing 5 mg/mL (100 mg/20 mL) of the antibody of the disclosure.

For administration to subjects, the appropriate volume of the antibody formulation is typically diluted in an infusion bag of for example 0.9% sodium chloride solution. The final infusion volume corresponding to the dose of the antibody of the disclosure is administered for a period of time that depends on the dose to be administered and thus on the protein amount given per hour.

In a specific embodiment, the anti-CD38 antibody for use according to the disclosure is administered as a naked antibody. The naked anti-CD38 antibody can be administered alone, or together with dexamethasone, or in combination with a chemo drug selected from the group consisting of lenalidomide, carfilzomib, bortezomib, melphalan, vincristine, cytarabine and cyclophosphamide, optionally together with dexamethasone. If not administered alone, the anti-CD38 antibody and the other drug(s) can be administered either simultaneously or separately (e.g. sequentially over a period of time).

The antibody of the disclosure may be administered in combination with a medication to prevent or control fatigue, nausea, pyrexia, cough, vomiting, hypercalcemia, headache, constipation, bone pain, chills, diarrhea, pneumonia, anemia, dysgeusia, hypokalemia, fever, and hyperglycemia.

In another embodiment, medication to prevent or control fatigue, nausea, pyrexia, cough, vomiting, hypercalcemia, headache, constipation, bone pain, chills, diarrhea, pneumonia, anemia, dysgeusia, hypokalemia, fever, and hyperglycemia may be administered prior to the antibody treatment.

In the context of the disclosure, a physician may evaluate the disease response and thus adapt the administration regime.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of").

Without limiting the present disclosure, a number of aspects of the present disclosure are further shown below for purpose of illustration:
Item 1: In one aspect, a method of treating a patient having relapsed and/or refractory multiple myeloma is disclosed which comprises administering to the patient an antibody that specifically binds CD38, wherein said antibody is administered to the patient in a safe therapeutic dose of 20 mg/kg or below.
Item 2: In another aspect, a pharmaceutical composition is disclosed which comprises an antibody that specifically binds CD38 for use as a medicament in the treatment of relapsed and/or refractory multiple myeloma, wherein said antibody is to be administered to a human subject in a safe therapeutic dose of about 20 mg/kg or less.
Item 3: In another easpect, disclosed here is the method or pharmaceutical composition according to Items 1 or 2, wherein the antibody is capable of killing a CD38⁺ cell in the human subject by induction of apoptosis, antibody-dependent cell-mediated cytotoxicity (ADCC), and complement-dependent cytotoxicity (CDC).
Item 4: In another aspect, also disclosed here is the method or pharmaceutical composition according any one of Items 1 to 3, wherein the patient has at least one condition selected from the group consisting of (a) measurable serum M-protein level greater than about 0.5 g/dL, (b) urine M-protein level greater than about 200 mg (24-hr urine), (c) elevated level of serum free light chains (FLC) greater than about 10 mg/dL with abnormal FLC ratio, and combination thereof.
Item 5: In another aspect, also disclosed here is the method or pharmaceutical composition according to any one of Items 1 to 4, wherein said antibody comprises at least one heavy chain and at least one light chain, wherein said heavy chain comprises three sequential complementarity-determining regions (CDRs) having amino acid sequences represented by SEQ ID NOS: 13, 37, and 15, and wherein said light chain comprises three sequential complementarity-determining regions (CDRs) having amino acid sequences represented by SEQ ID NOS: 16, 17, and 18.
Item 6: In another aspect, also disclosed here is the method or pharmaceutical composition according to any one of Items 1 to 5, wherein said antibody comprises at least one heavy chain comprising an amino acid sequence represented by SEQ ID NO: 50 and at least one light chain comprising an amino acid sequence represented by SEQ ID NO: 52.
Item 7: In another aspect, also disclosed here is the method or pharmaceutical composition according to any one of Items 1 to 6, wherein the safe therapeutic dose is from about 1 mg/kg to about 20 mg/kg.
Item 8: In another aspect, also disclosed here is the method or pharmaceutical composition according to any one of Items 1 to 6, wherein the safe therapeutic dose is about 5 mg/kg, or about 10 mg/kg, or about 20 mg/kg.
Item 9: In another aspect, also disclosed here is the method or pharmaceutical composition according to any one of Items 1 to 8, wherein the safe therapeutic dose of said antibody is administered intravenously.
Item 10: In another aspect, also disclosed here is the method or pharmaceutical composition according to any one of Items 1 to 9, wherein the safe therapeutic dose of said antibody is administered once a week or once every two weeks.
Item 11: In another aspect, also disclosed here is the method or pharmaceutical composition according to any one of Items 1 to 10, wherein the safe therapeutic dose is about 10 mg/kg or about 20 mg/kg administered once every two weeks.
Item 12: In another aspect, also disclosed here is the method or pharmaceutical composition according to any one of Items 1 to 10, wherein the safe therapeutic dose is about 10 mg/kg or about 20 mg/kg administered once every week.
Item 13: In another aspect, also disclosed here is the method or pharmaceutical composition according to any one of Items 1 to 12, wherein the safe therapeutic dose of said antibody is administered at an initial rate of infusion ranging from about 0.042 mg/hr to about 250 mg/hr.
Item 14: In another aspect, also disclosed here is the method or pharmaceutical composition according to any one of Items 1 to 13, wherein the antibody is administered in combination with dexamethasone.
Item 15: In another aspect, also disclosed here is the method or pharmaceutical composition according to any one of Items 1 to 14, wherein said antibody does not produce autoantibodies against said antibody when administered to a human subject at a dose of about 20 mg/kg or less.
Item 16: In another aspect, also disclosed here is the method or pharmaceutical composition according to any one of Items 1 to 10, wherein said antibody is capable of exhibiting detectable CD38 receptor occupancy in a human subject when administered to said human subject at a dose level of about 1 mg/kg every two weeks.
Item 17: In another aspect, also disclosed here is the method or pharmaceutical composition according to any one of Items 1 to 10, wherein said antibody is capable of exhibiting at least about 84.1% CD38 receptor occupancy in a human subject when administered to said human subject at a dose level of about 10 mg/kg or about 20 mg/kg every two weeks.
Item 18: In another aspect also disclosed here is the method or pharmaceutical composition according to any one of Items 1 to 10, wherein said antibody is capable of exhibiting at least about 97.7% CD38 receptor occupancy in a human subject when administered to said human subject at a dose level of about 10 mg/kg or about 20 mg/kg every two weeks.
Item 19: In another aspect, also disclosed here is the method or pharmaceutical composition according to any one of Items 1 to 10, wherein said antibody is capable of inhibiting tumor growth in a human subject when administered to said human subject at a dose level ranging from about 5 mg/kg to about 20 mg/kg every two weeks.
Item 20: In another aspect, also disclosed here is the method or pharmaceutical composition according to any one of Items 1 to 10, wherein said antibody is capable of inhibiting tumor growth in a human subject when administered to said human subject at a dose level ranging from about 5 mg/kg to about 20 mg/kg every week.
Item 21: In another aspect, also disclosed here is the method or pharmaceutical composition according to any one of Items 1 to 10, wherein said antibody is capable of inhibiting tumor growth in a human subject when administered to said human subject at a dose level ranging from about 10 mg/kg to about 20 mg/kg every two weeks.
Item 22: In another aspect, also disclosed here is the method or pharmaceutical composition according to any one of Items 1 to 10, wherein said antibody is capable of inhibiting tumor growth in a human subject when administered to said human subject at a dose level ranging from about 10 mg/kg to about 20 mg/kg every week.
Item 23: In another aspect, also disclosed here is a unit dosage form comprising the pharmaceutical composition of any one of Items 1 to 22.
Item 24: In another aspect, also disclosed here is an article of manufacture comprising the pharmaceutical composition of any one of Items 1 to 22 and a container.

### BRIEF DESCRIPTION OF THE SEQUENCES

SEQ ID NO: 1-3 shows the sequence of the CDR1-H, CDR2-H, CDR3-H of the "38SB13" antibody.
SEQ ID NO: 4-6 shows the sequence of the CDR1-L, CDR2-L, CDR3-L of the "38SB13" antibody.
SEQ ID NO: 7-9 shows the sequence of the CDR1-H, CDR2-H, CDR3-H of the "38SB18" antibody.
SEQ ID NO: 10-12 shows the sequence of the CDR1-L, CDR2-L, CDR3-L of the "38SB18" antibody.
SEQ ID NO: 13-15 shows the sequence of the CDR1-H, CDR2-H, CDR3-H of the "38SB19" antibody.
SEQ ID NO: 16-18 shows the sequence of the CDR1-L, CDR2-L, CDR3-L of the "38SB19" antibody.
SEQ ID NO: 19-21 shows the sequence of the CDR1-H, CDR2-H, CDR3-H of the "38SB30" antibody.
SEQ ID NO: 22-24 shows the sequence of the CDR1-L, CDR2-L, CDR3-L of the "38SB30" antibody.
SEQ ID NO: 25-27 shows the sequence of the CDR1-H, CDR2-H, CDR3-H of the "38SB31" antibody.
SEQ ID NO: 28-30 shows the sequence of the CDR1-L, CDR2-L, CDR3-L of the "38SB31" antibody.
SEQ ID NO: 31-33 shows the sequence of the CDR1-H, CDR2-H, CDR3-H of the "38SB39" antibody.
SEQ ID NO: 34-36 shows the sequence of the CDR1-L, CDR2-L, CDR3-L of the "38SB39" antibody.
SEQ ID NO: 37 shows the sequence of the CDR2-H of the humanized "38SB19" antibody. SEQ ID NO: 38- 43 shows the VL sequences of the antibodies.
SEQ ID NO: 44- 49 shows the VH sequences of the antibodies.
SEQ ID NO: 50- 51 shows the VH sequences of the humanized antibodies.
SEQ ID NO: 52-55 shows the VL sequences of the humanized antibodies.
SEQ ID NO: 56-61 shows the sequences of primers used for the for the first round degenerate PCR reaction for cloning and sequencing of the light and heavy chains of anti-CD38 antibodies.
SEQ ID NO: 62-63 shows the 5' end murine leader sequence primers used for the 38SB19 second round PCR reactions for cloning and sequencing of the light and heavy chains of anti-CD38 antibodies.

The present disclosure will be further illustrated by the following examples.

### EXAMPLE 1

The inventors determined for the antibody hu38SB19 the suitable dose of administration and regimen in order to obtain a well-tolerated anti-cancer treatment that enables treating patients suffering from a CD38⁺ hematological malignancy (see trial's information in Sanofi: *"*Dose escalation study of Anti-CD38 Monoclonal Antibody in patients with selected CD38+ Hematological malignancies", as available on 15 October 2013, ClinicalTrials Identifier: NCT01084252), in particular, from multiple Myeloma, more particularly from relapsed and/or refractory multiple Myeloma.

The following example thus discloses the results of the Phase 1 clinical trials demonstrating safe therapeutic dose of this specific anti-CD38 antibody hu38SB19 for an efficient treatment of CD38⁺ hematological malignancy such as multiple myeloma in humans.

### Patients

A total of 32 patients were treated in the study. 40.6% of the population were female; the mean age was 64.8 (±8.8). The Kornofsky status was greater than 60% in all patients.

The 32 patients included 3 patients with B-cell non-Hodgkins Lymphoma (NHL), 2 with chronic lymphocytic leukemia (CLL), and 27 with multiple myeloma (MM).

The 80.0% of the multiple myeloma patients were female with a mean age of 63.6 (±8.0). The multiple myeloma patients received as prior anti-cancer therapies bortezomib in 100%, lenalidomide in 92.6%, and autologous stem cell transplantation (ASCT) in 81.5% of the cases.

### Methods

The naked humanized IgG1 monoclonal antibody (mAb) hu38SB19 was administered as a single agent IV infusion every week (QW) or every 2 weeks (Q2W) to adult patients with selected CD38⁺ hematological malignancies who have progressed on or after standard therapy or for whom no effective standard therapy exists.

An accelerated dose escalation schedule was used for the first 5 dose levels (DL) (0.0001 mg/kg to 0.1 mg/kg every 2 weeks), with one evaluable patient per dose level unless the toxicity dose level (DLT) was experienced. All subsequent dose levels (0.3 mg/kg, 1 mg/kg, 3 mg/kg, 5mg/kg, 10 mg/kg, 20 mg/kg every 2 weeks and 10 mg/kg every week), followed the classic 3+3 design for dose escalation based on dose level toxicity.

The 32 patients were evaluable for dose level toxicity endpoint assessment and 32 for tumor response assessment.

The median number of cycles ranged from 2.0 to 50.0.

### Results

32 patients have been treated across all dose levels including 3 patients with B-cell non-Hodgkins Lymphoma (NHL), 2 with chronic lymphocytic leukemia (CLL), and 27 with multiple myeloma (MM).

The studies concerning dose levels of 20 mg/kg every 2 weeks and 10 mg/kg once a week dose are currently evaluated and the Maximum tolerated dose has not been reached.

Dose limiting toxicities have been limited to Grade 2 infusion reactions during cycle 1 with 1 at DL 0.3 mg/kg and 1 at DL 3.0 mg/kg. This was mitigated by the implementation of routine pretreatment with methylprednisone, diphenhydramine, ranitidine and acetaminophen.

The most frequent occurring adverse events (≥ 10%) at all dose levels, regardless of causality, are fatigue (46.9%), nausea (31.3%), pyrexia (28.1%), cough (25%), vomiting (21.9%), hypercalcemia (18.8%), with headache, constipation, bone pain, chills and diarrhea each occurring in 15.6% of patients. In addition, pneumonia, anemia, dysgeusia and hypokalemia each occurred in 12.5% of patients.

Serious adverse events that are related to therapy include Grade 3 pneumonia (6.3%) associated with fever (3.1%), hyperglycemia (3.1%) and one Grade 2 infusion reaction (3.1%).

Of the 19 patients treated at dose level 1.0 mg/kg to 10 mg/kg every 2 weeks, 1 had chronic lymphocytic leukemia (CLL), 1 had non-Hodgkins Lymphoma (NHL) and 17 had multiple myeloma (MM).

The 17 multiple myeloma patients were older and heavily pretreated patients; median age of 64 years (range: 55-74); and median of seven prior regimens (range: 2-14). All MM patients had received prior lenalidomide and bortezomib. The median time from diagnosis to first dosing with hu38SB19 was 6. 8 years (range 1.8 - 16.8 years).

Responses in this group (Figure 1), according to European Group for Bone Marrow and Transplant (EBMT) multiple myeloma criteria, included 1 partial response (PR) at 1 mg/kg (n = 3) and 5 mg/kg (n=3), and 1 minimal response (MR) at dose level 3 mg/kg (n = 6). The dose level 10 mg/kg demonstrated 3 partial responses (PR) and 2 stable disease (SD) among 6 multiple myeloma patients treated.

For the 19 patients treated at or above the 1 mg/kg DL the median time on treatment is 8 weeks (range 2-50 weeks).

Immunogenicity studies show no anti-hu38SB19 antibodies.

Receptor Occupancy could be detected from dose levels of 1 mg/kg and reached a range of 84.1 to 97.7 % at 10 mg/kg.

Pharmacokinetic analysis (PK) show a more than dose proportional increase of exposure over the 0.03 to 10 mg/kg dose range with clearance in a similar range between 5 mg/kg and 10 mg/kg.

No accumulation was observed based on Cₘₐₓ at cycle 2 over the 0.03 to 3 mg/kg dose range.

Tumor growth inhibition threshold was reached at Cₘₐₓ for 1 patient at DL 5 mg/kg and 5 patients at DL 10 mg/kg.

The anti-CD38 antibody hu38SB19 demonstrates encouraging single agent activity in patients with heavily pretreated relapsed and/or refractory multiple myeloma patients.

The disclosure may employ, unless otherwise indicated, conventional techniques of immunology, molecular biology and cell biology, which are well known in the art.

## Claims

1. A humanized monoclonal antibody that specifically binds CD38 for use in the treatment of relapsed and/or refractory multiple myeloma in a human subject, wherein:
said multiple myeloma is relapsed or refractory from previous treatment with lenalidomide and bortezomib;
said antibody comprises at least one heavy chain and at least one light chain;
said heavy chain comprises the amino acid sequence set forth in SEQ ID NO: 50 ;
said light chain comprises the amino acid sequence set forth in SEQ ID NO: 52;
said antibody is capable of killing a CD38+ cell in the human subject by induction of apoptosis, antibody-dependent cell-mediated cytotoxicity (ADCC), and complement-dependent cytotoxicity (CDC); and
said antibody is to be administered to a human subject in a safe therapeutic dose of 10mg/kg or 20 mg/kg once every two weeks.

2. The antibody for use according to claim 1, wherein the safe therapeutic dose of said antibody is for intravenous administration.

3. The antibody for use according to claim 1 or claim 2, wherein the safe therapeutic dose of said antibody is for administration by infusion at an initial rate of infusion ranging from 0.042 mg/hr to 250 mg/hr.

4. The antibody for use according to any one of claims 1 to 3, wherein said antibody does not produce autoantibodies against said antibody when administered to a human subject.

5. The antibody for use according to claim 1 or claim 2, wherein said antibody is capable of inhibiting tumor growth in a human subject when administered to said human subject at a dose level of 10 mg or 20 mg/kg every two weeks.

## Patentansprüche

1. Humanisierter monoklonaler Antikörper, der CD38 spezifisch bindet, zur Verwendung bei der Behandlung von rezidiviertem und/oder refraktärem multiplen Myelom bei einem humanen Individuum, wobei:
das multiple Myelom in Bezug auf eine vorherige Behandlung mit Lenalidomid und Bortezomib rezidiviert oder refraktär ist;
der Antikörper zumindest eine schwere Kette und zumindest eine leichte Kette umfasst;
die schwere Kette die in SEQ ID NO: 50 angeführte Aminosäuresequenz umfasst;
die leichte Kette die in SEQ ID NO: 52 angeführte Aminosäuresequenz umfasst;
der Antikörper in der Lage ist, eine CD38+-Zelle in dem humanen Individuum mittels Induktion von Apoptose, antikörperabhängiger zellvermittelter Zytotoxizität (ADCC) und komplementabhängiger Zytotoxizität (CDC) zu töten; und
der Antikörper einem humanen Individuum in einer sicheren therapeutischen Dosis von 10 mg/kg oder 20 mg/kg einmal alle zwei Wochen zu verabreichen ist.

2. Antikörper zur Verwendung nach Anspruch 1, wobei die sichere therapeutische Dosis des Antikörpers für eine intravenöse Verabreichung vorgesehen ist.

3. Antikörper zur Verwendung nach Anspruch 1 oder 2, wobei die sichere therapeutische Dosis des Antikörpers für eine Verabreichung durch Infusion mit einer anfänglichen Infusionsrate im Bereich von 0,042 mg/h bis 250 mg/h vorgesehen ist.

4. Antikörper zur Verwendung nach einem der Ansprüche 1 bis 3, wobei der Antikörper keine Autoantikörper gegen den Antikörper produziert, wenn er einem humanen Individuum verabreicht wird.

5. Antikörper zur Verwendung nach Anspruch 1 oder 2, wobei der Antikörper in der Lage ist, das Tumorwachstum bei einem humanen Individuum zu hemmen, wenn es dem humanen Individuum in einer Dosismenge von 10 mg oder 20 mg/kg alle zwei Wochen verabreicht wird.

## Revendications

1. Anticorps monoclonal humanisé qui se lie spécifiquement à CD38 destiné à être utilisé dans le traitement du myélome multiple en rechute et/ou réfractaire chez un sujet humain, dans lequel :
ledit myélome multiple est en rechute ou réfractaire par rapport à un traitement précédent par le lénalidomide et le bortézomib ;
ledit anticorps comprend au moins une chaîne lourde et au moins une chaîne légère ;
ladite chaîne lourde comprend la séquence d'acides aminés décrite dans SEQ ID NO: 50 ;
ladite chaîne légère comprend la séquence d'acides aminés décrite dans SEQ ID NO: 52 ;
ledit anticorps est capable de tuer une cellule CD38+ chez le sujet humain par l'induction d'une apoptose, d'une cytotoxicité à médiation cellulaire dépendante des anticorps (ADCC), et d'une cytotoxicité dépendante du complément (CDC) ; et
ledit anticorps doit être administré à un sujet humain en une dose thérapeutique sûre de 10 mg/kg ou 20 mg/kg une fois toutes les deux semaines.

2. Anticorps destiné à être utilisé selon la revendication 1, dans lequel la dose thérapeutique sûre dudit anticorps est pour une administration intraveineuse.

3. Anticorps destiné à être utilisé selon la revendication 1 ou la revendication 2, dans lequel la dose thérapeutique sûre dudit anticorps est pour une administration par perfusion à un débit de perfusion initial compris entre 0,042 mg/h et 250 mg/h.

4. Anticorps destiné à être utilisé selon l'une quelconque des revendications 1 à 3, où ledit anticorps ne produit pas d'auto-anticorps dirigés contre ledit anticorps lorsqu'il est administré à un sujet humain.

5. Anticorps destiné à être utilisé selon la revendication 1 ou la revendication 2, où ledit anticorps est capable d'inhiber la croissance tumorale chez un sujet humain lorsqu'il est administré audit sujet humain à une dose de 10mg ou 20 mg/kg toutes les deux semaines.
